# EUROPEAN PATENT APPLICATION

(11) **EP 4 708 307 A1**
(43) Date of publication of application: **11.03.2026**
(21) Application number: 25199319.2
(22) Date of filing: 01.09.2025
(51) Int. Cl.: G16C 20/80, G16B 40/10, G16B 45/00, G16C 20/20

(54) **MOLECULAR NETWORK FOR LIBRARY SPECTRAL CONTENT**

(30) Priority: 09.09.2024 US 202418828491
(71) Applicant: Thermo Finnigan LLC, San Jose, CA 95134 (US); HighChem s.r.o., 821 09 Bratislava (SK); Thermo Fisher Scientific S.p.A, 20054 Segrate (MI) (IT)
(72) Inventor: STRATTON, Timothy James, Austin (US); RAAB, Michal, Bratislava (SK); DURICA, Ondrej, Dunajska Luzna (SK); ULASZEWSKA-TARANTINO, Maria Malgorzata, Milan (IT); BODNÁR, Gergo, Bratislava (SK); MEZEY, Jakub, Bratislava (SK)
(74) Representative: Boult Wade Tennant LLP

(57) **Abstract**

Embodiments described herein relate to a process for molecular network generation. A system can comprise a memory that stores, and a processor that executes, computer executable components. The computer executable components can comprise an evaluating component that executes a comparison of first spectrum data to second spectrum data, a scoring component that, based on the comparison, generates a spectrum similarity score describing a level of similarity of the first spectrum data to the second spectrum data, a parameterizing component that, based on the comparison, associates a first secondary property corresponding to the first spectrum data with the second spectrum data or associates a second secondary property corresponding to the second spectrum data with the first spectrum data, and a generating component that generates a grouping of spectral data comprising the first spectrum data and the second spectrum data based on the spectrum similarity score and on the associating.

## Description

The subject patent application is related to U.S. Patent Application No. __, filed September 9, 2024, and entitled "MOLECULAR NETWORK FOR LIBRARY MOLECULAR STRUCTURAL CONTENT" (attorney docket no. TP387483USPRV1_TFSP137US), the entirety of which is hereby incorporated by reference herein.

### BACKROUND

A molecular network can be employed to exploit an assumption that structurally related molecules can produce similar fragmentation patterns, and therefore can be notated as related within the molecular network. Such molecular network can be used to address a high capacity of library content that increases over time.

### BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 illustrates a block diagram of an example scientific instrument for performing one or more operations, in accordance with one or more embodiments described herein.
FIG. 2 illustrates a flow diagram of an example method of performing operations using the scientific instrument of FIG. 1, in accordance with one or more embodiments described herein.
FIG. 3 illustrates a graphical user interface (GUI) that can be used in the performance of one or more of the methods described herein, in accordance with one or more embodiments described herein.
FIG. 4 illustrates a block diagram of an example computing device that can perform one or more of the methods disclosed herein, in accordance with one or more embodiments described herein.
FIG. 5 illustrates a block diagram of an example, non-limiting system that can facilitate a process for molecular network generation and/or visualization, in accordance with one or more embodiments described herein.
FIG. 6 illustrates a block diagram of another example, non-limiting system that can facilitate a process for molecular network generation and/or visualization, in accordance with one or more embodiments described herein.
FIG. 7 illustrates an exemplary molecular network visualization, in accordance with one or more embodiments described herein.
FIG. 8 illustrates another exemplary molecular network visualization, in accordance with one or more embodiments described herein.
FIG. 9 illustrates a diagram of an interactive GUI that can be employed to customize one or more parameters employed by the one or more embodiments described herein to generate a molecular network visualization, in accordance with one or more embodiments described herein.
FIG. 10 illustrates a flow diagram of one or more processes that can be performed by the molecular network generation system of FIG. 5, in accordance with one or more embodiments described herein.
FIG. 11 illustrates another flow diagram of one or more processes that can be performed by the molecular network generation system of FIG. 6, in accordance with one or more embodiments described herein.
FIG. 12 illustrates a continuation of the flow diagram of FIG. 11 of one or more processes that can be performed by the molecular network generation system of FIG. 6, in accordance with one or more embodiments described herein.
FIG. 13 illustrates a flow diagram of one or more processes that can be performed by the molecular network generation system of FIG. 5, in accordance with one or more embodiments described herein.
FIG. 14 illustrates another flow diagram of one or more processes that can be performed by the molecular network generation system of FIG. 6, in accordance with one or more embodiments described herein.
FIG. 15 illustrates a continuation of the flow diagram of FIG. 14 of one or more processes that can be performed by the molecular network generation system of FIG. 6, in accordance with one or more embodiments described herein.
FIG. 16 illustrates a block diagram of example scientific instrument system in which one or more of the methods described herein can be performed, in accordance with one or more embodiments described herein.
FIG. 17 illustrates a block diagram of an example operating environment into which embodiments of the subject matter described herein can be incorporated.
FIG. 18 illustrates an example schematic block diagram of a computing environment with which the subject matter described herein can interact and/or be implemented at least in part.

### SUMMARY

The following presents a summary to provide a basic understanding of one or more embodiments described herein. This summary is not intended to identify key or critical elements, and/or to delineate scope of particular embodiments or scope of claims. Its sole purpose is to present concepts in a simplified form as a prelude to the more detailed description that is presented later. In one or more embodiments, systems, computer-implemented methods, apparatuses and/or computer program products described herein can provide a plug-and-play process for generating, visualizing and/or employing a molecular network for various databases using a visualization framework.

In accordance with an embodiment, a system can comprise a memory that stores computer executable components, and a processor that executes the computer executable components. The computer executable components can comprise an evaluating component that executes a comparison of first spectrum data to second spectrum data, a scoring component that, based on the comparison, generates a spectrum similarity score describing a level of similarity of the first spectrum data to the second spectrum data, a parameterizing component that, based on the comparison, associates a first secondary property corresponding to the first spectrum data with the second spectrum data or associates a second secondary property corresponding to the second spectrum data with the first spectrum data, and a generating component that generates a grouping of spectral data comprising the first spectrum data and the second spectrum data based on the spectrum similarity score and on the associating.

In accordance with another embodiment, a computer-implemented method can comprise executing, by a system operatively coupled to a processor, a comparison of first spectrum data to second spectrum data, based on the comparison, generating, by the system, a spectrum similarity score describing a level of similarity of the first spectrum data to the second spectrum data, based on the comparison, associating, by the system, a first secondary property corresponding to the first spectrum data with the second spectrum data or associating, by the system, a second secondary property corresponding to the second spectrum data with the first spectrum data, and generating, by the system, a grouping of spectral data comprising the first spectrum data and the second spectrum data based on the spectrum similarity score and on the associating.

In accordance with still another embodiment, a computer program product facilitates a process for generation of one or more spectral data groupings, the program instructions executable by a processor to cause the processor to execute, by the processor, a comparison of first spectrum data to second spectrum data, based on the comparison, generate, by the processor, a spectrum similarity score describing a level of similarity of the first spectrum data to the second spectrum data, based on the comparison, associate, by the processor, a first secondary property corresponding to the first spectrum data with the second spectrum data or associate, by the processor, a second secondary property corresponding to the second spectrum data with the first spectrum data, and generate, by the processor, a grouping of spectral data comprising the first spectrum data and the second spectrum data based on the spectrum similarity score and on the associating.

The one or more embodiments described herein can employ a novel system that provides for limited error (e.g., few to one data points of unknown data) being employed when updating a spectral library and generating a molecular network therefrom. In this way, by using known spectral data, and gradually building out the spectral data, unknown spectra can be classified and/or identified, while limiting compounded errors during the generating (e.g., as compared to existing frameworks that employ plural unknown data points when generating an update to a spectral library for an unknown compound).

The one or more embodiments described herein can be implemented within, in connection with and/or coupled to a scientific imaging device.

The one or more embodiments disclosed herein can be applied on a plug-and-play basis to various architectures of existing spectral library and/or library datastores of spectral data. That is, the one or more embodiments described herein can generate a molecular network comprising a visual representing a plurality of chemical relationships regardless of data structure of a spectral library.

In one or more embodiments described herein, a spectral data grouping can be generated from a molecular network and provided as any one or more of a visual, data, metadata, etc. The spectral data grouping can be generated based on one or more of a) one or more similarity scores between pairs of spectrum data or b) one or more secondary properties of at least one of the spectral data of the pairs of spectrum data. That is, in one or more embodiments, a spectral data grouping can be based on similarity scores and on a secondary property. In one or more other embodiments, a spectral data grouping can be based on a first secondary property and at least one other secondary property.

The one or more embodiments described herein can provide the molecular network visual being a dynamically adjustable visual that can provide varied visualization types and/or customization of visualized chemical relationships and/or properties. For example, dynamic adjustability can be found in functioning of the generated molecular network (MN), where a user entity can interact with the visual display to vary illustrated chemical classes, chemical properties, sizes and/or distances of varying MN aspects, etc. Varied visualizations can comprise large MN clouds, customized clouds based on one or more specified parameters, plural clouds displayed at a same time as one another, etc. Customization can be provided by use of a graphical user interface (GUI) allowing for different chemical properties and/or relationships to be represented by nodes, edges, borders of nodes and/or edges, fill of nodes and/or edges, thickness of lines within a cloud, distances between nodes, etc.

The one or more embodiments described herein can be employed to generate a molecular network that can provide varying outputs during use of the molecular network. For example, based on visual aspects of a format of a MN cloud, such as coloring, line thicknesses, shapes and/or distances between different aspects of the MN cloud, a user entity, and/or the system itself, can predict one or more chemical properties and/or relationships corresponding to an unknown spectra. These one or more chemical properties and/or relationships can comprise chemical class, chemical use, similar compounds, etc.

Further aspects of the invention are set out in the following numbered clauses:
Clause 1. A system, comprising:
   a memory that stores computer executable components; and
   a processor that executes the computer executable components stored in the memory, wherein the computer executable components comprise:
      an evaluating component that executes a comparison of first spectrum data to second spectrum data;
      a scoring component that, based on the comparison, generates a spectrum similarity score describing a level of similarity of the first spectrum data to the second spectrum data;
      a parameterizing component that, based on the comparison, associates a first secondary property corresponding to the first spectrum data with the second spectrum data or associates a second secondary property corresponding to the second spectrum data with the first spectrum data; and
      a generating component that generates a grouping of spectral data comprising the first spectrum data and the second spectrum data based on the spectrum similarity score and on the associating.
Clause 2. The system of clause 1, wherein the grouping of spectral data comprises a dataset or data employed to generate a visualization.
Clause 3. The system of clause 1, wherein the associated one of the first secondary property or the second secondary property is defined by identification metadata associated with the first spectrum data or the second spectrum data.
Clause 4. The system of clause 1, wherein the scoring component generates the spectrum similarity score describing a comparison of a first mass to charge ratios of ions of the first spectrum data to a second mass to charge ratios of ions of the second spectrum data.
Clause 5. The system of clause 1, wherein the first spectrum data is a first unknown spectrum data, and wherein the second spectrum data is a second unknown spectrum data.
Clause 6. The system of clause 1, wherein the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.
Clause The system of clause 1, wherein the computer executable components further comprise:
   a displaying component that displays a visual, at a graphical user interface, comprising an edge, corresponding to the spectrum similarity score, extending between a pair of nodes, corresponding to a first spectrum defined by the first spectrum data and a second spectrum defined by the second spectrum data.
Clause 8. The system of clause 7, wherein the computer executable components further comprise:
   a parameterizing component that applies a first property of the spectrum similarity score as a first visual modification of the edge and that applies the associated one of the first secondary property or the second secondary property as a second visual modification of the respective node of the first spectrum or of the second spectrum.
Clause The system of clause 8, wherein the parameterizing component adjusts at least one of the first visual modification or the second visual modification based on selection, at a graphical user interface comprising the visual, from a class of properties comprising properties other than at least one of the first property or the second property.
Clause 10. A computer-implemented method, comprising:
   executing, by a system operatively coupled to a processor, a comparison of first spectrum data to second spectrum data;
   based on the comparison, generating, by the system, a spectrum similarity score describing a level of similarity of the first spectrum data to the second spectrum data;
   based on the comparison, associating, by the system, a first secondary property corresponding to the first spectrum data with the second spectrum data or associating, by the system, a second secondary property corresponding to the second spectrum data with the first spectrum data; and
   generating, by the system, a grouping of spectral data comprising the first spectrum data and the second spectrum data based on the spectrum similarity score and on the associating.
Clause 11. The computer-implemented method of clause 10, wherein the grouping of spectral data comprises a dataset or data employed to generate a visualization.
Clause 12. The computer-implemented method of clause 10, wherein the associated one of the first secondary property or the second secondary property is defined by identification metadata associated with the first spectrum data or the second spectrum data.
Clause 13. The computer-implemented method of clause 10, further comprising:
   generating, by the system, the spectrum similarity score describing a comparison of a first mass to charge ratios of ions of the first spectrum data to a second mass to charge ratios of ions of the second spectrum data.
Clause 14. The computer-implemented method of clause 10, wherein the first spectrum data is a first unknown spectrum data, and wherein the second spectrum data is a second unknown spectrum data.
Clause 15. The computer-implemented method of clause 10, wherein the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.
Clause 16. A computer program product facilitating a process for generation of one or more spectral data groupings, the computer program product comprising a computer readable storage medium having program instructions embodied therewith, and the program instructions executable by a processor to cause the processor to:
   execute, by the processor, a comparison of first spectrum data to second spectrum data;
   based on the comparison, generate, by the processor, a spectrum similarity score describing a level of similarity of the first spectrum data to the second spectrum data;
   based on the comparison, associate, by the processor, a first secondary property corresponding to the first spectrum data with the second spectrum data or associate, by the processor, a second secondary property corresponding to the second spectrum data with the first spectrum data; and
   generate, by the processor, a grouping of spectral data comprising the first spectrum data and the second spectrum data based on the spectrum similarity score and on the associating.
Clause 17. The computer program product of clause 16, wherein the grouping of spectral data comprises a dataset or data employed to generate a visualization.
Clause 18. The computer program product of clause 16, wherein the associated one of the first secondary property or the second secondary property is defined by identification metadata associated with the first spectrum data or the second spectrum data.
Clause 19. The computer program product of clause 16, wherein the first spectrum data is a first unknown spectrum data, and wherein the second spectrum data is a second unknown spectrum data.
Clause 20. The computer program product of clause 16, wherein the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.

### DETAILED DESCRIPTION

The following detailed description is merely illustrative and is not intended to limit embodiments and/or application or utilization of embodiments. Furthermore, there is no intention to be bound by any expressed or implied information presented in the preceding Summary section, or in the Detailed Description section. One or more embodiments are now described with reference to the drawings, wherein like reference numerals are utilized to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

Various operations can be described as multiple discrete actions or operations in turn, in a manner that is most helpful in understanding the subject matter disclosed herein. However, the order of description should not be construed as to imply that these operations are necessarily order dependent. In particular, these operations can be performed in an order different from the order of presentation. Operations described can be performed in a different order from the described embodiment. Various additional operations can be performed, and/or described operations can be omitted in additional embodiments.

Turning now to the subject of molecular networking, molecular networking can organize spectral data, such as mass spectroscopy/mass spectroscopy (MS/MS) data as a relational network, such as a relational spectral network, thereby mapping the chemistry behind fragmentation patterns of chemicals. In existing frameworks, such molecular networking can be employed in untargeted metabolomics experiments to find structurally related metabolites in experimental datasets.

However, analysis of untargeted metabolomics datasets can be limited by the ability to annotate and identify metabolites. A rate of successful annotations in an untargeted dataset is usually very low. Curated spectral databases of analytical standards can be reference-point-employed in matching of fragmentation patterns. While calculating similarity scores for a hit can comprise an automated process, subsequent determining and selecting of a best candidate is a challenging step that suffers from difficulties in investigation of long lists of hits or time consuming examination of mirror plots for a plurality of, such as dozens of, highly ranked proposals.

Indeed, in cases of existing frameworks, this difficulty can be exacerbated by generation of a molecular network, and similarity scores or other relationships corresponding thereto, based on plural unknown inputs. This can include comparison of unknown spectra to a plurality of unknown spectra, determinations of relationships between unknown spectra, etc. This use of plural unknown datapoints to generate new data can result in the new data having errors built upon errors (e.g., compounded), thus leading to subsequent identification failures and/or other queries related to use of a molecular network generated by existing frameworks.

Further, in existing frameworks, a spectrum search analysis can calculate hundreds of scores for analytical standards exhibiting structural similarity to query spectrum. However, the examination of long tables can be a difficult, error-prone and/or time-consuming task. For example, hits belonging to a same chemical family or class are frequently placed at different positions of a corresponding results table, making the interpretation difficult. A number of results also is typically high, making the evaluation process time consuming.

To account for one or more deficiencies of such existing frameworks, one or more embodiments are described herein that can provide rapid increase and efficiency of selection of a nearest chemically-related analytical standard and/or speed up an associated annotation process corresponding to a molecular network (MN). In one or more cases, one or more embodiments described herein can speed up annotation generation processes, resulting in more efficient and/or rapidly performed spectrum search analyses.

Generally, the one or more embodiments described herein can employ a novel system that provides for limited error (e.g., few to one data points of unknown data) being employed when updating a spectral library and generating a molecular network therefrom. In this way, by using known spectral data, and gradually building out the spectral data, unknown spectra can be classified and/or identified, while limiting compounded errors during the generating (e.g., as compared to existing frameworks that employ plural unknown data points when generating an update to a spectral library for an unknown compound).

Additionally, and/or alternatively, the one or more embodiments described herein can employ the novel system to provide greater accuracy and/or more specific spectral data groupings to further limit unusable spectral data that is returned based on a query and/or based on one or more parameter adjustments and/or filtering adjustments performed by and/or requested by a user entity. For example, in one or more embodiments described herein, a spectral data grouping can be generated from a molecular network and provided as any one or more of a visual, data, metadata, etc. The spectral data grouping can be generated based on one or more of a) one or more similarity scores between pairs of spectrum data or b) one or more secondary properties of at least one of the spectral data of the pairs of spectrum data. That is, in one or more embodiments, a spectral data grouping can be based on similarity scores and on a secondary property. In one or more other embodiments, a spectral data grouping can be based on a first secondary property and at least one other secondary property.

That is, the one or more embodiments described herein can provide generation of a molecular network based on a spectral library, updating of the spectral library based on an unknown spectra, generation of a dynamically-adjustable and customizable molecular network cloud visual, and/or generation of a classification output based on the molecular network cloud visual.

The one or more embodiments described herein can provide the molecular network visual being a dynamically adjustable visual that can provide varied visualization types and/or customization of visualized chemical relationships and/or properties. For example, dynamic adjustability can be found in functioning of the generated molecular network (MN), where a user entity can interact with the visual display to vary chemical classes, chemical properties, sizes and/or distances of varying MN aspects, etc. Varied visualizations can comprise large MN clouds, customized clouds based on one or more specified parameters, plural clouds displayed at a same time as one another, etc. Customization can be provided by use of a graphical user interface (GUI) allowing for different chemical properties and/or relationships to be represented by nodes, edges, borders of nodes and/or edges, fill of nodes and/or edges, thickness of lines within a cloud, distances between nodes, etc.

One or more benefits can comprise comparison of unknown spectrum with molecular network of highly curated spectral trees with different metadata taxonomies, simultaneous visualization of plural nearest network families exhibiting structural relationships with a query spectrum, facilitation of a decision making process to correctly judge and select highly scored best hits, and/or exploitation of chemical diversity of chemical entities present in a library, underlying the molecular network, and comprising different chemical property and/or chemical relationship filtering options.

For example, one or more molecular networking application embodiments as described herein can aid in determining and/or selecting one or more best hits from spectrum similarity search results. In one or more cases, such one or more embodiments can enhance understanding of structural similarities between query and library through visualization of nodes and edges that can comprise different metadata available in various libraries and/or library types. The specificity and accuracy of spectral data groupings provided by the one or more embodiments described here cannot be provided by existing frameworks. Rather, the one or more frameworks described herein can provide for any one or more of parameter adjustments, filtering, similarity score determinations, etc., where any one or more of such aspects can be employed in combination to generate, by the one or more frameworks, one or more spectra data groupings (e.g., data, metadata, visual or non-visual).

That is, the one or more embodiments described herein can be employed to generate a molecular network that can provide varying outputs during use of the molecular network. For example, based on visual aspects of a format of a MN cloud, such as coloring, line thicknesses, shapes and/or distances between different aspects of the MN cloud, a user entity, or the system itself, can predict one or more chemical properties and/or relationships corresponding to an unknown spectrum. These one or more chemical properties and/or relationships can comprise chemical class, chemical use, similar compounds, etc.

Also using the one or more embodiments described herein, a molecular network can be explored without querying a particular spectrum. By generating an efficient standalone network of relationships, such as similarities of a library, the one or more embodiments described herein can aid in exploration and/or browsing of content of a library, and in one or more cases, visualization of chemical diversity of the library.

Further, regarding functioning of the one or more embodiments described herein, such can be implemented within, in connection with and/or coupled to a scientific imaging device. This implementation can be applied on a plug-and-play basis to various architectures of existing spectral library and/or library datastores of spectral data. That is, the one or more embodiments described herein can generate a molecular network comprising a visual representing a plurality of chemical relationships regardless of data structure of a spectral library.

Discussion next turns to a general discussion of one or more scientific instrument systems disclosed herein, as well as to related methods, computing devices, and/or computer-readable media. For example, in one or more embodiments, a system can comprise a memory that stores computer executable components and a processor that executes the computer executable components stored in the memory. The computer executable components can comprise an evaluating component that executes a comparison of first spectrum data, comprising first mass to charge ratios of ions exhibited at an unknown spectrum, to second spectrum data, comprising second mass to charge ratios of ions exhibited at a known analytical spectrum of a library of known analytical spectra, and an updating component that applies an update to the library of known analytical spectra based on the comparison of the unknown spectrum to the known analytical spectrum.

The one or more embodiments disclosed herein can achieve improved performance relative to existing approaches, as noted above. For example, based on application of a use of a single unknown data point and known spectral data of a specified spectra library, at least a portion of a MN (e.g., a relationship, spectral similarity score, etc.) can be generated, allowing for generation of a visual MN cloud comprising this portion of the MN. Use of the one or more molecular network generating frameworks discussed herein can allow for generation and display of a dynamically-adjustable and customizable molecular network cloud and/or a determination of a classification related to an unknown spectrum query (e.g., the single unknown data point).

The embodiments disclosed herein thus can provide improvements to scientific instrument technology (e.g., improvements in the computer technology supporting such scientific instruments, among other improvements), which can be employed in various fields including optics, signal processing, spectroscopy, and/or nuclear magnetic resonance (NMR), without being limited thereto.

Various ones of the embodiments disclosed herein can improve upon existing approaches to achieve the technical advantages of high information and/or accurate information molecular network generation, visualization and/or operation (e.g., use of the MN). That is, the one or more frameworks described herein can provide a more accurate construction, as compared to existing frameworks, of a molecular network and/or molecular network cloud visual based on the MN, thereby allowing for identification of a chemical property, chemical relationship, and/or chemical classification for an unknown spectrum query. This identification can be based on data/metadata underlying the MN generated and/or on the visual representation of the corresponding MN cloud. The unknown spectral data query can originally arise from any suitable source, such as from a scientific imaging device source using any suitable method, such as high-performance liquid chromatography (HPLC), gas chromatography (GC), ion chromatography (IC), HPLC-mass spectroscopy (HPLC-MS), GC-mass spectroscopy (GC-MS), IC-mass spectroscopy (IC-MS), nuclear magnetic resonance (NMR), raman spectroscopy, infrared spectroscopy, and/or the like, without being limited thereto.

Such technical advantages are not achievable by routine and/or existing approaches, as described above, and all user entities of systems including such embodiments can benefit from these advantages (e.g., by assisting the user entity in the performance of a technical task, such as identification of one or more unknown compound queries, by means of molecular network generation, molecular network visualization, and/or molecular network operation.

The technical features of the embodiments disclosed herein (e.g., analysis of data defining an unknown spectra absent use of additional unknown data points) is thus decidedly unconventional in the field of material analysis, in addition to the fields of optics, signal processing, spectroscopy, and/or NMR, without being limited thereto, as are combinations of the features of the embodiments disclosed herein.

As discussed further herein, various aspects of the embodiments disclosed herein can improve the functionality of a computer itself. That is, the computational and/or user interface features disclosed herein do not involve only the collection and/or comparison of information but instead can apply new analytical and technical techniques to change the operation of the computer-analysis of material compounds. For example, based on the generation of a molecular network (e.g., employing a single point of unknown data per addition of unknown spectrum query), a MN having greater accuracy, reduced compound error, and/or faster use for determining queries can be provided, as compared to existing frameworks. As a result thereof, use of a MN and/or of a MN visualization (e.g., MN cloud visual) can be accompanied by an increase in speed and/or accuracy of response related to a query. As such, one or more non-limiting systems described herein, comprising a molecular network generation system, can be self-improving.

The present disclosure thus introduces functionality that neither an existing computing device, nor a human, could perform. Rather, such existing computing devices are ineffective at generation of molecular networks, relationships are poorly represented, and/or the examination of long tables can be a difficult, error-prone and/or time-consuming task in view of compounded errors and poor relationship representation, thereby resulting in loss of accuracy, efficiency and/or speed when submitting a query to such existingly-generated MNs. In view of the time, energy and/or loss of data involved, it is not practical to operate within the confines of existing approaches.

Accordingly, the embodiments of the present disclosure can serve any of a number of technical purposes, such as controlling a specific technical system or process; determining from measurements how to control a machine; digital audio, image, or video enhancement or analysis; separation of material sources in a mixed signal; generating data for reliable and/or efficient transmission or storage; providing estimates and confidence intervals for material samples; or providing a faster processing of sensor data. In particular, the present disclosure provides technical solutions to technical problems, including, but not limited to, hologram modification; image/signal blurring; application of combined blurring techniques; and/or subsequent image reconstruction, resulting in a faster, more thorough and/or more efficient processing of generated images and thus of material samples or other target compositions being imaged.

The embodiments disclosed herein thus provide improvements to material analysis technology (e.g., improvements in the computer technology supporting material analysis, among other improvements).

As used herein, the phrase "based on" should be understood to mean "based at least in part on," unless otherwise specified.

As used herein, the term "component" can refer to an atomic element, molecular element, phase of an atomic or molecular element, or combination thereof.

As u sed herein, the term "compound" can refer to a single material, multiple materials, composition, sample, solution, product, etc.

As used herein, the term "data" can comprise metadata.

As used herein, the terms "entity," "requesting entity," and "user entity" can refer to a machine, device, component, hardware, software, smart device, party, organization, individual and/or human.

One or more embodiments are now described with reference to the drawings, where like referenced numerals are used to refer to like drawing elements throughout. In the following description, for purposes of explanation, numerous specific details are set forth in order to provide a more thorough understanding of the one or more embodiments. It is evident in various cases, however, that the one or more embodiments can be practiced without these specific details.

Further, it should be appreciated that the embodiments depicted in one or more figures described herein are for illustration only, and as such, the architecture of embodiments is not limited to the systems, devices and/or components depicted therein, nor to any particular order, connection and/or coupling of systems, devices and/or components depicted therein.

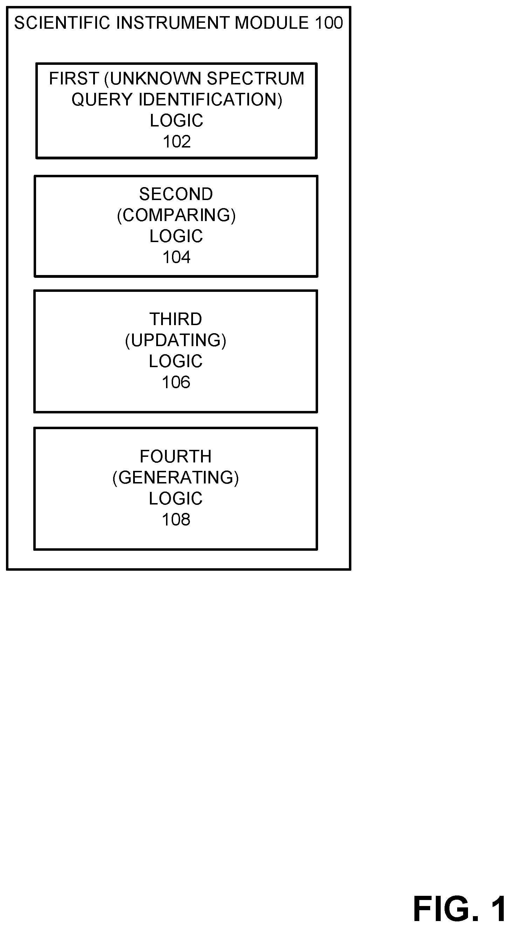

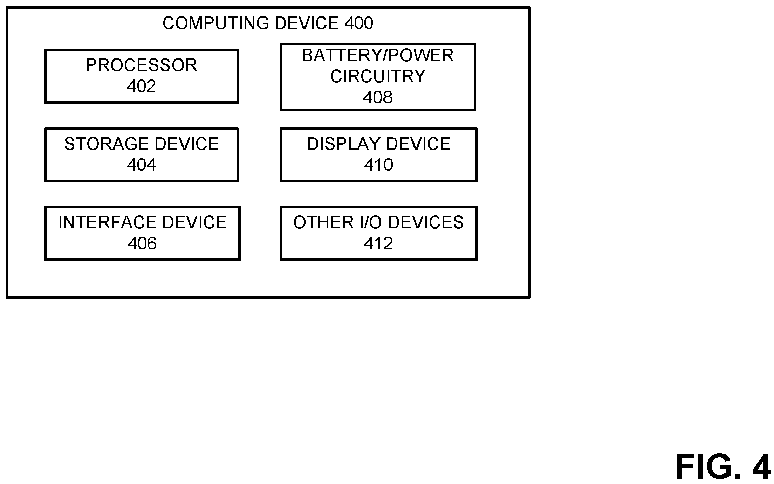

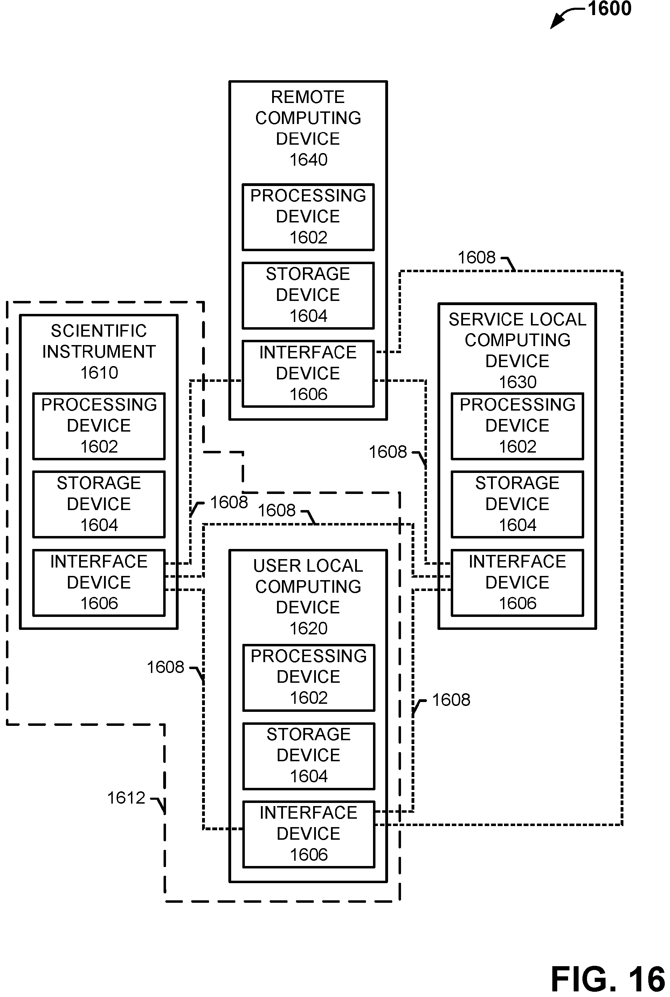

Turning now in particular to the one or more figures, and first to FIG. 1, illustrated is a block diagram of a scientific instrument module 100 for performing material analysis operations using a molecular network generation and/or visualization process, in accordance with various embodiments described herein. The scientific instrument module 100 can be implemented by circuitry (e.g., including electrical and/or optical components), such as a programmed computing device. The logic of the scientific instrument module 100 can be included in a single computing device or can be distributed across multiple computing devices that are in communication with each other as appropriate. Examples of computing devices that can, singly or in combination, implement the scientific instrument module 100 are discussed herein with reference to the computing device 400 of FIG. 4, and examples of systems of interconnected computing devices, in which the scientific instrument module 100 can be implemented across one or more of the computing devices, is discussed herein with reference to the scientific instrument system 1600 of FIG. 16.

The scientific instrument module 100 can include first logic 102, second logic 104, third logic 106, and fourth logic 108. As used herein, the term "logic" can include an apparatus that is to perform a set of operations associated with the logic. For example, any of the logic elements included in the module 100 can be implemented by one or more computing devices programmed with instructions to cause one or more processing devices of the computing devices to perform the associated set of operations. In a particular embodiment, a logic element can include one or more non-transitory computer-readable media having instructions thereon that, when executed by one or more processing devices of one or more computing devices, cause the one or more computing devices to perform the associated set of operations. As used herein, the term "module" can refer to a collection of one or more logic elements that, together, perform a function associated with the module. Different ones of the logic elements in a module can take the same form or can take different forms. For example, some logic in a module can be implemented by a programmed general-purpose processing device, while other logic in a module can be implemented by an application-specific integrated circuit (ASIC). In another example, different ones of the logic elements in a module can be associated with different sets of instructions executed by one or more processing devices. A module can omit one or more of the logic elements depicted in the associated drawing; for example, a module can include a subset of the logic elements depicted in the associated drawing when that module is to perform a subset of the operations discussed herein with reference to that module.

The first logic 102 can receive, find, locate, download, request, measure and/or otherwise determine data and/or metadata defining an unknown spectrum query. That is, the first logic 102 can obtain data for being processed and for subsequent use in generating a molecular network cloud visual or updating a spectral library.

The second logic 104 can perform a comparing process by generally comparing an unknown spectrum to a known analytical spectrum of a library data store (e.g., spectral library), absent comparison of the unknown spectrum to another unknown spectrum. That is, the second logic 104 can employ the output of the first logic 102 as a trigger for the second logic 104.

The third logic 106 can update the spectral library based on the comparison of the second logic 104. That is, the third logic 106 can employ an output of the second logic 104 to perform the third logic 106.

The fourth logic 108 can generate a spectral data grouping, such as comprising data and/or metadata, and/or comprising a molecular network (MN) cloud visual and/or other data representing a MN cloud visual. The spectral data grouping can comprise and/or is based on the update, and thus on the comparison. That is, the fourth logic 108 can generate the spectral data grouping based on the execution of the third logic 106.

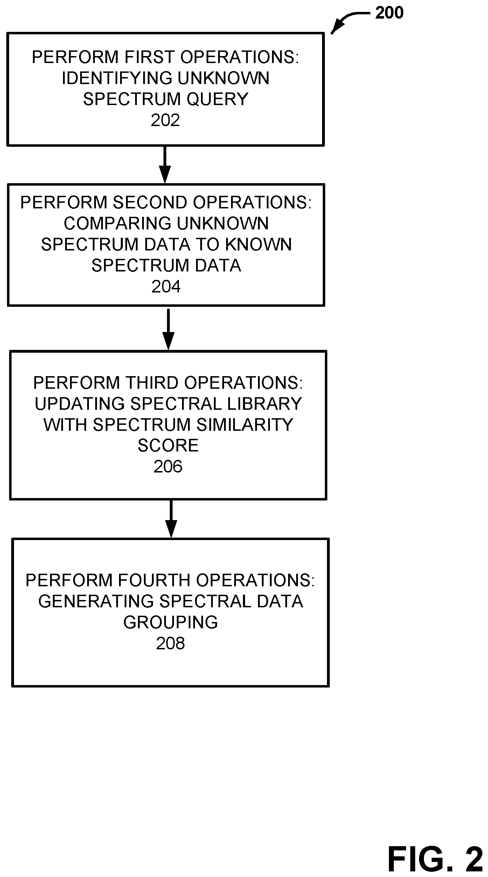

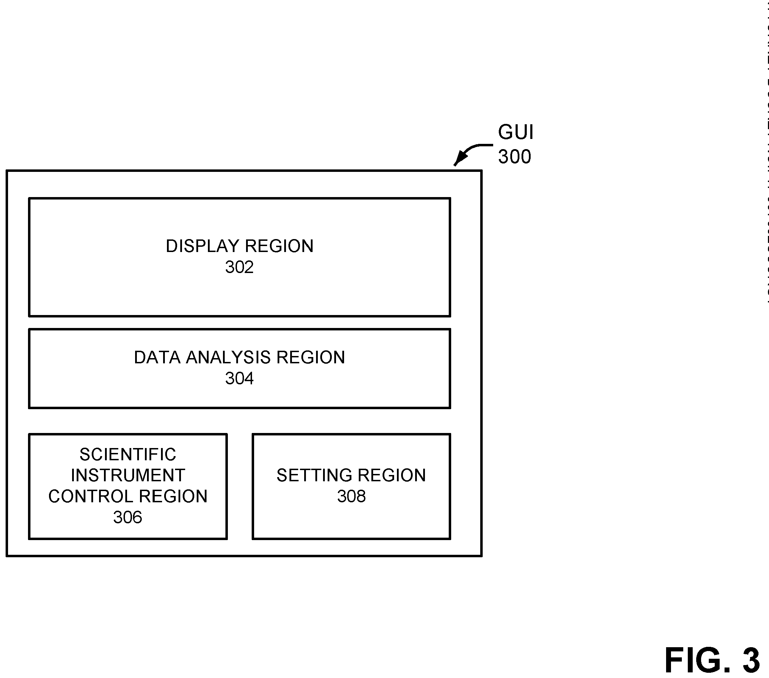

FIG. 2 illustrates a flow diagram of a method 200 of performing operations, by the scientific instrument module 100, in accordance with various embodiments. Although the operations of the method 200 can be illustrated with reference to particular embodiments disclosed herein (e.g., the scientific instrument module 100 discussed herein with reference to FIG. 1, the GUI 300 discussed herein with reference to FIG. 3, the computing device 400 discussed herein with reference to FIG. 4, and/or the scientific instrument system 1600 discussed herein with reference to FIG. 16), the method 200 can be used in any suitable setting to perform any suitable operations. Operations are illustrated once each and in a particular order in FIG. 2, but the operations can be reordered and/or repeated as desired and appropriate (e.g., different operations performed can be performed in parallel, as suitable).

At 202, first operations can be performed. For example, the first logic 102 of the module 100 can perform the first operations 202. The first operations 202 can include receiving, finding, locating, downloading, requesting, measuring and/or otherwise determining data and/or metadata defining the unknown spectrum query.

At 204, second operations can be performed. For example, the second logic 104 of the module 100 can perform the second operations 204. The second operations 204 can comprise comparing one or more properties of the unknown spectrum, such as a first mass to charge ratio of ions exhibited threat, to one or more properties of the known spectrum, such as a second mass to charge ratio of ions exhibited threat.

At 206, third operations can be performed. For example, the third logic 106 of the module 100 can perform the third operations 206. The third operations 206 can comprise updating a spectral library, such as by a write action adding data to define the unknown spectrum and/or a result of the comparison output from the second operations 204.

At 208, fourth operations can be performed. For example, the fourth logic 108 of the module 100 can perform the fourth operations 208. The fourth operations 208 can comprise generation of data/metadata defining a spectral data grouping, such as a MN cloud visual, based on the spectral library, and comprising a representation of the comparison output from the second operations 204, as updated into the spectral library by the third operations 206.

The scientific instrument methods disclosed herein can include interactions with a user entity (e.g., via the user local computing device 1620 discussed herein with reference to FIG. 16). These interactions can include providing information to the user entity (e.g., information regarding the operation of a scientific instrument such as the scientific instrument 1610 of FIG. 16, information regarding a sample being analyzed or other test or measurement performed by a scientific instrument, information retrieved from a local or remote database, or other information) or providing an option for a user entity to input commands (e.g., to control the operation of a scientific instrument such as the scientific instrument 1610 of FIG. 16, or to control the analysis of data generated by a scientific instrument), queries (e.g., to a local or remote database), or other information. In some embodiments, these interactions can be performed through a graphical user interface (GUI) that includes a visual display on a display device (e.g., the display device 410 discussed herein with reference to FIG. 4) that provides outputs to the user entity and/or prompts the user entity to provide inputs (e.g., via one or more input devices, such as a keyboard, mouse, trackpad, or touchscreen, included in the other I/O devices 412 discussed herein with reference to FIG. 4). The scientific instrument system 1600 disclosed herein can include any suitable GUIs for interaction with a user entity.

Turning next to FIG. 3, depicted is an example GUI 300 that can be used in the performance of one or more of the methods described herein, in accordance with various embodiments described herein. As noted above, the GUI 300 can be provided on a display device (e.g., the display device 410 discussed herein with reference to FIG. 4) of a computing device (e.g., the computing device 400 discussed herein with reference to FIG. 4) of a scientific instrument system (e.g., the scientific instrument system 1600 discussed herein with reference to FIG. 16), and a user entity can interact with the GUI 300 using any suitable input device (e.g., any of the input devices included in the other I/O devices 412 discussed herein with reference to FIG. 4) and input technique (e.g., movement of a cursor, motion capture, facial recognition, gesture detection, voice recognition, actuation of buttons, etc.).

The GUI 300 can include a data display region 302, a data analysis region 304, a scientific instrument control region 306, and a settings region 308. The particular number and arrangement of regions depicted in FIG. 3 is merely illustrative, and any number and arrangement of regions, including any desired features thereof, can be included in a GUI 300.

The data display region 302 can display data generated by a scientific instrument (e.g., the scientific instrument 1610 discussed herein with reference to FIG. 16). For example, the data display region 302 can display one or more output results which can comprise one or more spectra, one or more spectrum similarity scores, one or more cloud visuals and/or one or more cloud visual parameter control GUIs, without being limited thereto.

The data analysis region 304 can display the results of data analysis (e.g., the results of analyzing the data illustrated in the data display region 302 and/or other data). For example, the data analysis region 304 can display one or more of the output results of a query (e.g., an unknown compound query, spectral data grouping, etc.), such as a classification defining the unknown compound. In one or more cases, the data analysis region 304 can display a list, flow chart or other schematic of acquisition actions taken and/or recommended relative to an experiment. In one or more embodiments, the data display region 302 and the data analysis region 304 can be combined in the GUI 300 (e.g., to include data output from a scientific instrument, and some analysis of the data, in a common graph or region).

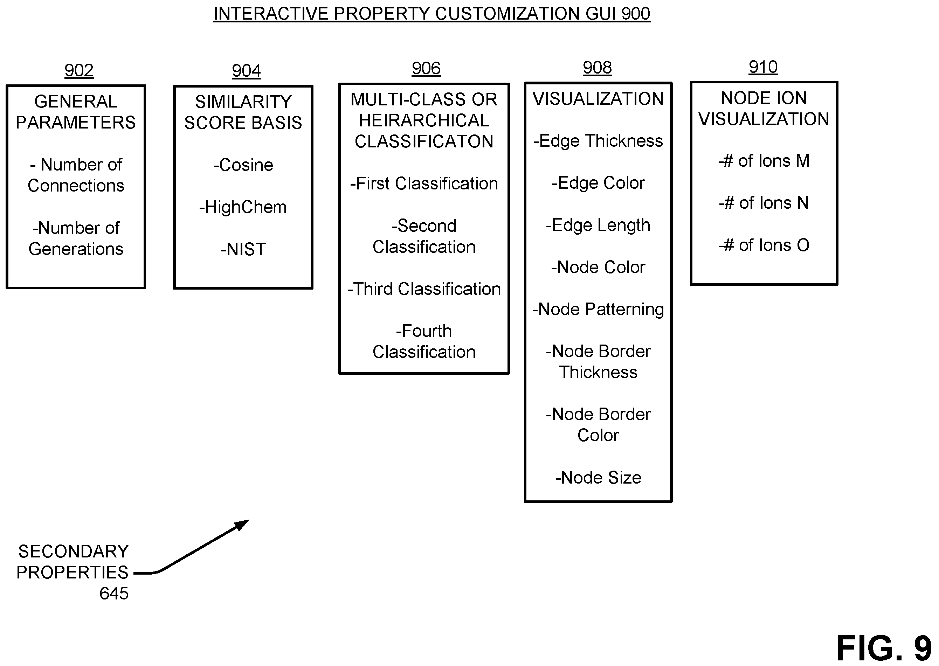

The scientific instrument control region 306 can include options that allow the user entity to control a scientific instrument (e.g., the scientific instrument 1610 discussed herein with reference to FIG. 16). For example, the scientific instrument control region 306 can include one or more controls for customizing a cloud visual, such as based on the GUI 900 of FIG. 9, to be described below.

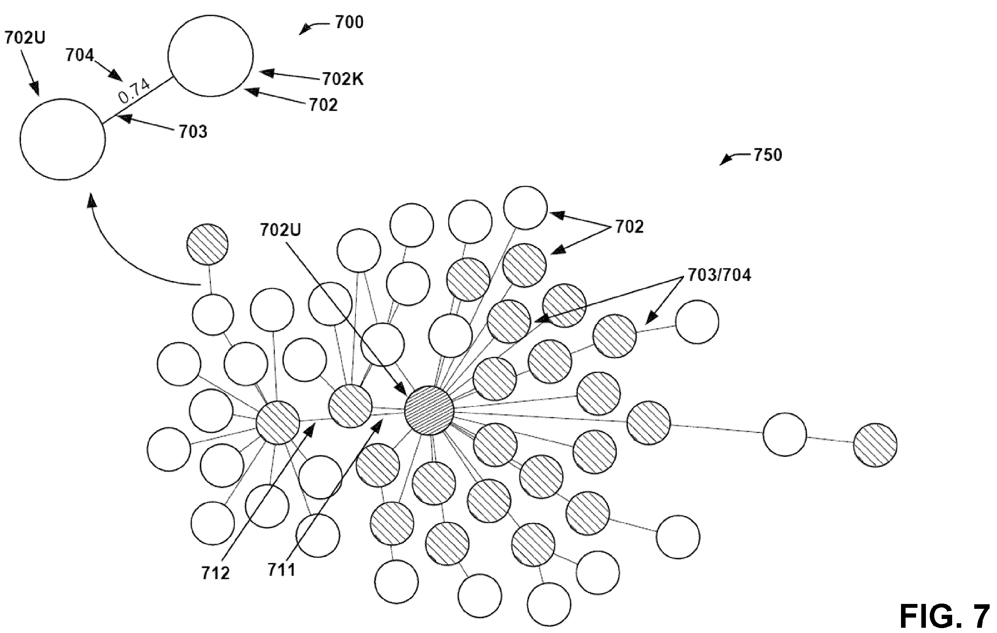

The settings region 308 can include options that allow the user entity to control the features and functions of the GUI 300 (and/or other GUIs) and/or perform common computing operations with respect to the data display region 302 and data analysis region 304 (e.g., saving data on a storage device, such as the storage device 404 discussed herein with reference to FIG. 4, sending data to another user entity, labeling data, etc.). For example, the settings region 308 can include one or more options to alter color, fill or format of illustrations, such as an illustration of any aspect of FIGS. 7-9 and/or other image, whether actual, representative and/or schematic, to be described below.

As noted above, the scientific instrument module 100 can be implemented by one or more computing devices. Accordingly, discussion next turns to FIG. 4, which illustrates a block diagram of a computing device 400 that can perform some or all of the scientific instrument methods disclosed herein, in accordance with various embodiments. In one or more embodiments, the scientific instrument module 100 can be implemented by a single computing device 400 or by multiple computing devices 400. Further, as discussed below, a computing device 400 (or multiple computing devices 400) that implements the scientific instrument module 100 can be part of one or more of the scientific instrument 1610, the user local computing device 1620, the service local computing device 1630, or the remote computing device 1640 of FIG. 16.

The computing device 400 of FIG. 4 is illustrated as having a number of components, but any one or more of these components can be omitted or duplicated, as suitable for the application and setting. As illustrated, these components can include one or more of a processor 402, storage device 404, interface device 406, battery/power circuitry 408, display device 410 and other input/output (I/O) devices 412, as will be described below.

In one or more embodiments, one or more of the components included in the computing device 400 can be attached to one or more motherboards and enclosed in a housing (e.g., including plastic, metal, and/or other materials). In one or more embodiments, some these components can be fabricated onto a single system-on-a-chip (SoC) (e.g., an SoC can include one or more processors 402 and one or more storage devices 404). Additionally, in one or more embodiments, the computing device 400 can omit one or more of the components illustrated in FIG. 4. In one or more embodiments, the computing device 400 can include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface). For example, the computing device 400 can omit a display device 410, but can include display device interface circuitry (e.g., a connector and driver circuitry) to which a display device 410 can be coupled.

The computing device 400 can include the processor 402 (e.g., one or more processing devices). As used herein, the term "processing device" can refer to any device or portion of a device that processes electronic data from registers and/or memory to transform that electronic data into other electronic data that can be stored in registers and/or memory. The processor 402 can include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 400 can include a storage device 404 (e.g., one or more storage devices). The storage device 404 can include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In one or more embodiments, the storage device 404 can include memory that shares a die with a processor 402. In such an embodiment, the memory can be used as cache memory and can include embedded dynamic random-access memory (eDRAM) or spin transfer torque magnetic random-access memory (STT-MRAM), for example. In one or more embodiments, the storage device 404 can include non-transitory computer readable media having instructions thereon that, when executed by one or more processing devices (e.g., the processor 402), cause the computing device 400 to perform any appropriate ones of or portions of the methods disclosed herein.

The computing device 400 can include an interface device 406 (e.g., one or more interface devices 406). The interface device 406 can include one or more communication chips, connectors, and/or other hardware and software to govern communications between the computing device 400 and other computing devices. For example, the interface device 406 can include circuitry for managing wireless communications for the transfer of data to and from the computing device 400. The term "wireless" and its derivatives can be used to describe circuits, devices, systems, methods, techniques, communications channels, etc., that can communicate data through the use of modulated electromagnetic radiation through a nonsolid medium. The term does not imply that the associated devices do not contain any wires, although in one or more embodiments the associated devices might not contain any wires. Circuitry included in the interface device 406 for managing wireless communications can implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In one or more embodiments, circuitry included in the interface device 406 for managing wireless communications can operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In one or more embodiments, circuitry included in the interface device 406 for managing wireless communications can operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In one or more embodiments, circuitry included in the interface device 406 for managing wireless communications can operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In one or more embodiments, the interface device 406 can include one or more antennas (e.g., one or more antenna arrays) to receipt and/or transmission of wireless communications.

In one or more embodiments, the interface device 406 can include circuitry for managing wired communications, such as electrical, optical, or any other suitable communication protocols. For example, the interface device 406 can include circuitry to support communications in accordance with Ethernet technologies. In one or more embodiments, the interface device 406 can support both wireless and wired communication, and/or can support multiple wired communication protocols and/or multiple wireless communication protocols. For example, a first set of circuitry of the interface device 406 can be dedicated to shorter-range wireless communications such as Wi-Fi or Bluetooth, and a second set of circuitry of the interface device 406 can be dedicated to longer-range wireless communications such as global positioning system (GPS), EDGE, GPRS, CDMA, WiMAX, LTE, EV-DO, or others. In one or more embodiments, a first set of circuitry of the interface device 406 can be dedicated to wireless communications, and a second set of circuitry of the interface device 406 can be dedicated to wired communications.

The computing device 400 can include battery/power circuitry 408. The battery/power circuitry 408 can include one or more energy storage devices (e.g., batteries or capacitors) and/or circuitry for coupling components of the computing device 400 to an energy source separate from the computing device 400 (e.g., AC line power).

The computing device 400 can include a display device 410 (e.g., multiple display devices). The display device 410 can include any visual indicators, such as a heads-up display, a computer monitor, a projector, a touchscreen display, a liquid crystal display (LCD), a light-emitting diode display, or a flat panel display.

The computing device 400 can include other input/output (I/O) devices 412. The other I/O devices 412 can include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 400, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 400 can have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

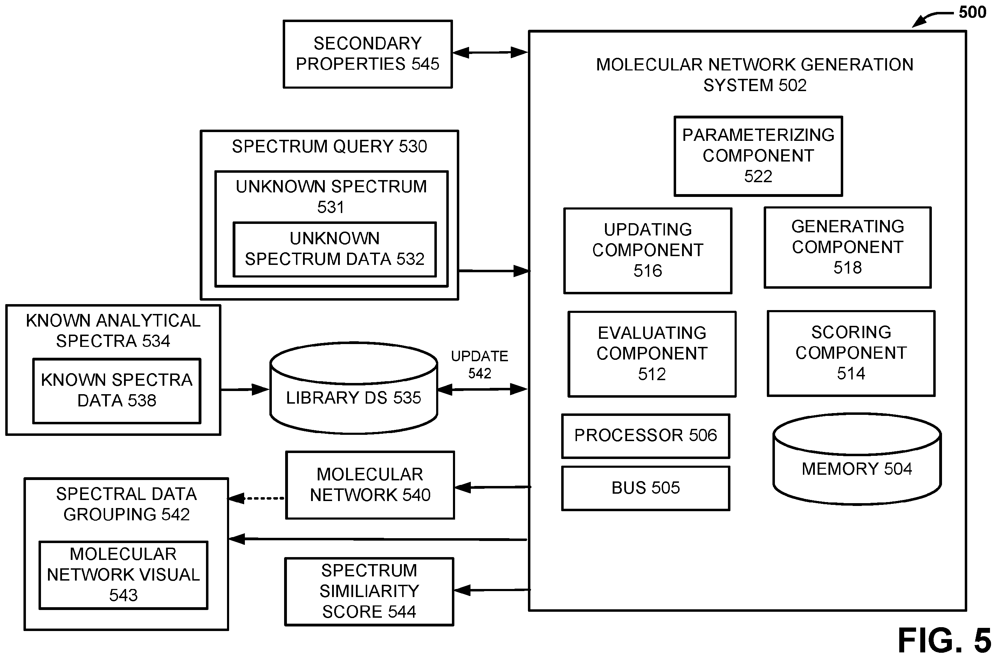

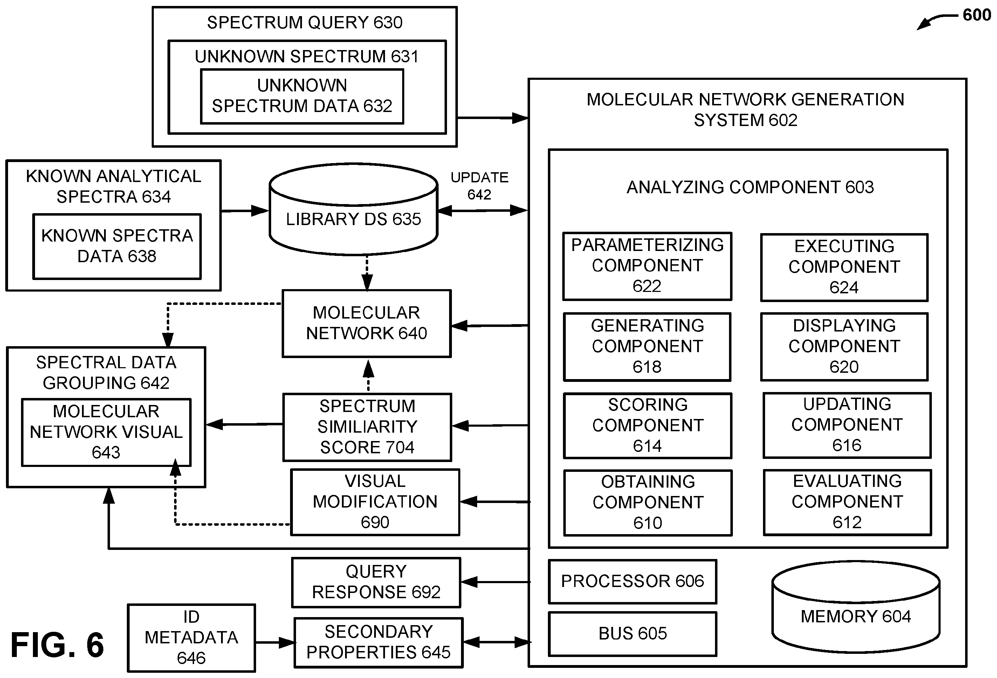

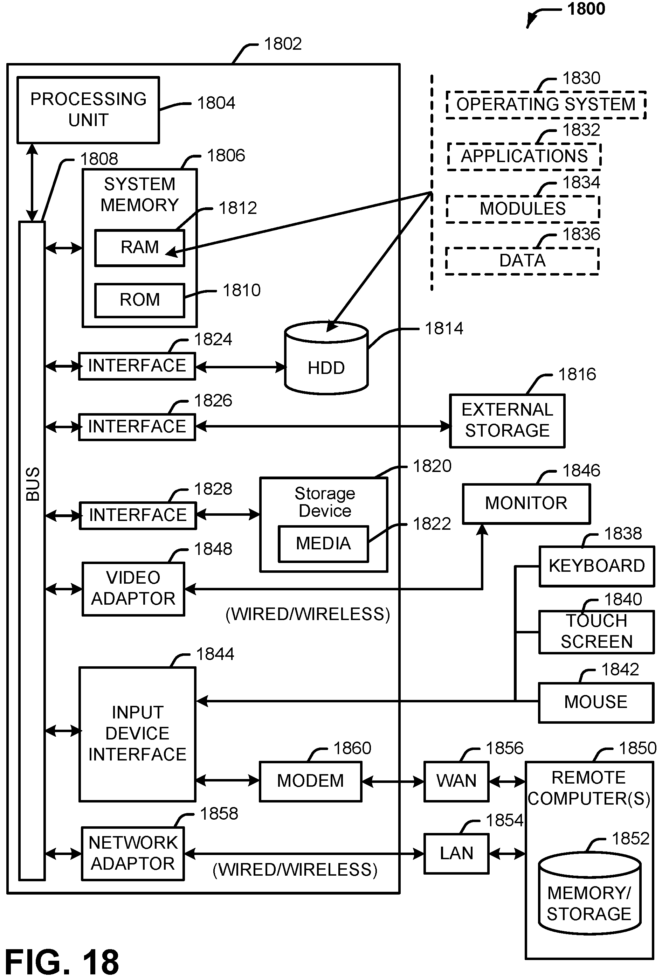

Referring now to FIGS. 5 and 6, in one or more embodiments, the non-limiting systems 500 and/or 600 illustrated at FIGS. 5 and 6, and/or systems thereof, can further comprise one or more computer and/or computing-based elements described herein with reference to a computing environment, such as the computing environment 1800 illustrated at FIG. 18. In one or more described embodiments, computer and/or computing-based elements can be used in connection with implementing one or more of the systems, devices, components and/or computer-implemented operations shown and/or described in connection with FIGS. 5 and/or 6 and/or with other figures described herein.

Turning first to FIG. 5, the figure illustrates a block diagram of an example, non-limiting system 500 that can comprise a molecular network generation system 502 and a library datastore (DS) 535. The molecular network generation system 502 can generally facilitate generation of a molecular network 540 via updating of the molecular network 540 (e.g., via an update 542) and/or generation of one or more spectral data groupings 542, such as comprised by data and/or metadata, and/or comprising a molecular network visual 543.

In one or more embodiments, the molecular network generation system 502 can be at least partially comprised by the computing device 400.

It is noted that the molecular network generation system 502 is only briefly detailed to provide but a lead-in to a more complex and/or more expansive molecular network generation system 602 as illustrated at FIG. 6. That is, further detail regarding processes that can be performed by one or more embodiments described herein will be provided below relative to the non-limiting system 600 of FIG. 6.

Still referring to FIG. 5, the molecular network generation system 502 can comprise at least a memory 504, bus 505, processor 506, evaluating component 512, scoring component 514, updating component 516, generating component 418 and/or parameterizing component 522. The processor 506 can be the same as the processor 402, comprised by the processor 402 or different therefrom. The memory 504 can be the same as the storage device 404, comprised by the storage device 404 or different therefrom.

Any one or more of the evaluating component 512, scoring component 514, updating component 516, generating component 418 and/or parameterizing component 522 can be operatively coupled to the processor 506 which can be operatively coupled to the memory 504. The bus 505 can provide for the operative coupling. The processor 506 can facilitate execution of the evaluating component 512, scoring component 514, updating component 516, generating component 418 and/or parameterizing component 522. Any one or more of the evaluating component 512, scoring component 514, updating component 516, generating component 418 and/or parameterizing component 522 can be stored at the memory 504.

In general, the non-limiting system 500 can employ any suitable method of communication (e.g., electronic, communicative, internet, infrared, fiber, etc.) to provide communication between the molecular network generation system 502 and/or any device associated with a user entity.

Turning now to a first embodiment based on the non-limiting system 500, the evaluating component 512 can execute a comparison of first spectrum data (e.g., unknown spectrum data 532), comprising first mass to charge ratios of ions exhibited at an unknown spectrum 531, to second spectrum data (e.g., known spectrum data 538), comprising second mass to charge ratios of ions exhibited at a known analytical spectrum 534 of a library of known analytical spectra (e.g., a spectral library or library datastore 535).

The scoring component 514 can generally determine whether there is another known spectrum (e.g., known analytical spectrum 534) against which to compare the unknown spectrum 531.

The updating component 516 generally can apply an update 542 to the spectral library (e.g., library datastore 535) based on the comparison of the unknown spectrum 531 to the known analytical spectrum 534.

As a result of these components, the molecular network 540 can be updated based on a single point of unknown data (e.g., the unknown spectrum data 532), absent comparison of the unknown spectrum data 532 to any additional unknown spectrum data. As discussed above, this can aid in limiting compounding of errors and reduction of accuracy in generation of the molecular network 540, where the generation can include the updating. Put another way, the molecular network generation system 502 can facilitate a process to at least partially generate the molecular network (MN) 540, such as by updating the library datastore 535, and thus the molecular network 540 that employs the library datastore 535, based on an update 542.

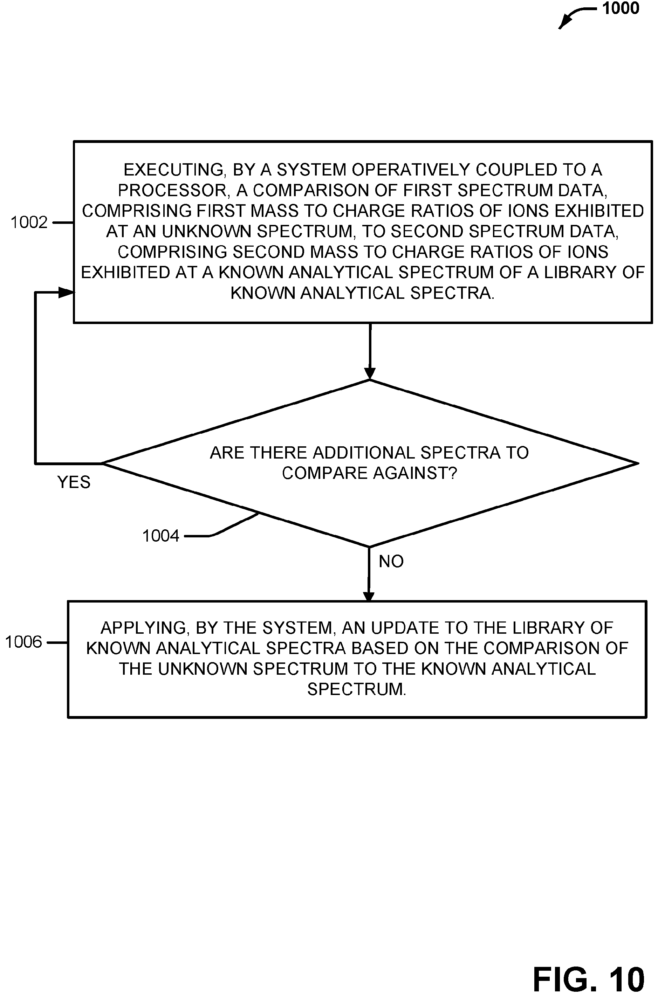

As a summary of the above-described components and functions thereof, referring next only briefly to FIG. 10, illustrated is a flow diagram of an example, non-limiting method 1000 that can facilitate a process to generate and/or update a MN, in accordance with one or more embodiments described herein, such as the non-limiting system 500 of FIG. 5. While the non-limiting method 1000 is described relative to the non-limiting system 500 of FIG. 5, the non-limiting method 1000 can be applicable also to other systems described herein, such as the non-limiting system 600 of FIG. 6. Repetitive description of like elements and/or processes employed in respective embodiments is omitted for sake of brevity.

At 1002, the non-limiting method 1000 can comprise executing, by the system (e.g., evaluating component 512), a comparison of first spectrum data (e.g., unknown spectrum data 532 and/or known spectrum data 538), comprising first mass to charge ratios of ions exhibited at an unknown spectrum (e.g., unknown spectrum 531), to second spectrum data (e.g., known spectrum data 538), comprising second mass to charge ratios of ions exhibited at a known analytical spectrum (e.g., known analytical spectra 534) of a library (e.g., library datastore 535) of known analytical spectra.

At 1004, the non-limiting method 1000 can comprise determining, by the system (e.g., scoring component 514), whether there are additional known spectra of the library against which to compare the unknown spectrum. If yes, the non-limiting method 1100 can proceed back to step 1002. If not, the non-limiting method can proceed forward to step 1006.

At 1006, the non-limiting method 1000 can comprise applying, by the system (e.g., updating component 516) an update to the library of known analytical spectra based on the comparison of the unknown spectrum to the known analytical spectrum.

Turning now to a second embodiment based on the non-limiting system 500, the evaluating component 512 can execute a comparison of first spectrum data to second spectrum data. The first spectrum data can be an unknown spectrum data 532 or a known spectrum data 538. The second spectrum data can be an unknown spectrum data 532 or a known spectrum data 538. For example, two unknown spectra data 532 can be compared, two known spectra data 538 can be compared, and/or an unknown spectrum data 532 and a known spectrum data 538 can be compared. As noted above, a known spectra data 538 can be obtained from a library datastore 535 or any other datastore of information employed by a molecular network 540 at least partially supported by the molecular network generation system 502.

The scoring component 514 can, based on the comparison performed by the evaluating component 512, generate a spectrum similarity score 544 describing a level of similarity of the first spectrum data to the second spectrum data.

The parameterizing component 522 can, based on the comparison, associate a first secondary property 545 corresponding to the first spectrum data with the second spectrum data or associate a second secondary property 545 corresponding to the second spectrum data with the first spectrum data.

The generating component 518 can generate a grouping of spectral data (e.g., spectral data grouping 542) comprising the first spectrum data and the second spectrum data. The spectral data grouping 542 can be based on the spectrum similarity score 544 and on the associating of the first secondary property 545 or the second secondary property 545.

As a result, a spectral data grouping 542 can be generated from a molecular network 540 and provided as any one or more of a visual (e.g., molecular network visual 543), data, metadata, etc. The spectral data grouping 542 can be generated based on one or more of a) one or more similarity scores 544 between pairs of spectrum data 532, 538 or b) one or more secondary properties 545 of at least one of the spectral data 532, 538 of the pairs of spectrum data. That is, in one or more embodiments, a spectral data grouping 542 can be based on similarity scores 544 and on a secondary property 545. In one or more other embodiments, a spectral data grouping 542 can be based on a first secondary property 545 and at least one other secondary property 545.

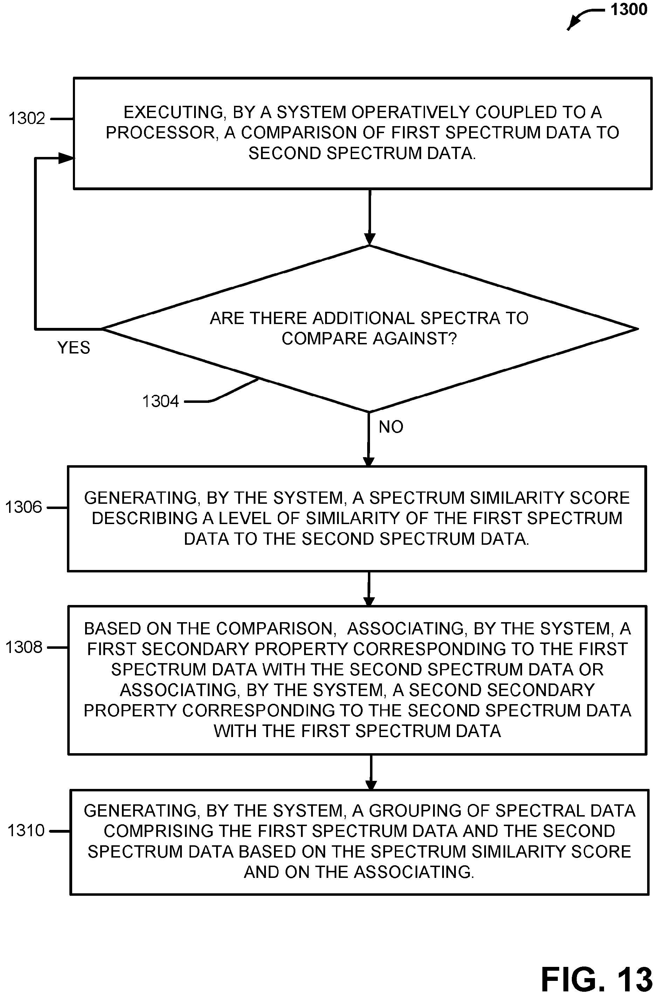

As another summary of the above-described components and functions thereof, referring next only briefly to FIG. 13, illustrated is a flow diagram of an example, non-limiting method 1300 that can facilitate a process to generate and/or update a spectral data grouping from a molecular network, in accordance with one or more embodiments described herein, such as the non-limiting system 500 of FIG. 5. While the non-limiting method 1300 is described relative to the non-limiting system 500 of FIG. 5, the non-limiting method 1300 can be applicable also to other systems described herein, such as the non-limiting system 600 of FIG. 6. Repetitive description of like elements and/or processes employed in respective embodiments is omitted for sake of brevity.

At 1302, the non-limiting method 1300 can comprise executing, by the system (e.g., evaluating component 512), a comparison of first spectrum data (e.g., unknown spectrum data 532 or known spectrum data 538) to second spectrum data (e.g., unknown spectrum data 532 or known spectrum data 538).

At 1304, the non-limiting method 1300 can comprise determining, by the system (e.g., scoring component 514), whether there are additional known spectra of a library (e.g., library datastore 535) against which to compare the unknown spectrum. If yes, the non-limiting method 1300 can proceed back to step 1302. If not, the non-limiting method can proceed forward to step 1306.

At 1306, the non-limiting method 1300 can comprise generating, by the system (e.g., scoring component 514), a spectrum similarity score (e.g., spectrum similarity score 544) describing a level of similarity of the first spectrum data to the second spectrum data.

At 1308, the non-limiting method 1300 can comprise, based on the comparison, associating, by the system (e.g., parameterizing component 522), a first secondary property (e.g., secondary property 545) corresponding to the first spectrum data with the second spectrum data or associating, by the system (e.g., parameterizing component 522), a second secondary property (e.g., secondary property 545) corresponding to the second spectrum data with the first spectrum data.

At 1310, the non-limiting method 1300 can comprise generating, by the system (e.g., generating component 518), a grouping of spectral data comprising the first spectrum data and the second spectrum data based on the spectrum similarity score and on the associating.

Turning next to FIG. 6, a non-limiting system 600 is illustrated that can comprise a molecular network generation system 602 and a library datastore (DS) 635. Repetitive description of like elements and/or processes employed in respective embodiments is omitted for sake of brevity. Description relative to an embodiment of FIG. 5 can be applicable to an embodiment of FIG. 6. Likewise, description relative to an embodiment of FIG. 6 can be applicable to an embodiment of FIG. 5.

Generally, the molecular network generation system 602 can facilitate a process to at least partially generate the molecular network (MN) 640, such as by updating the library datastore 635, and thus the molecular network 640 that employs the library datastore 635, based on an update 642. In one or more embodiments, the MN generation system 602 further can facilitate a process to generate and/or display a spectral data grouping 642, such as molecular network visual 643, such as a MN cloud visual 750 (see, e.g., FIG. 7, to be discussed below).

In one or more embodiments, the molecular network generation system 602 can be at least partially comprised by the computing device 400.

One or more communications between one or more components of the non-limiting system 600 can be provided by wired and/or wireless means including, but not limited to, employing a cellular network, a wide area network (WAN) (e.g., the Internet), and/or a local area network (LAN). Suitable wired or wireless technologies for supporting the communications can include, without being limited to, wireless fidelity (Wi-Fi), global system for mobile communications (GSM), universal mobile telecommunications system (UMTS), worldwide interoperability for microwave access (WiMAX), enhanced general packet radio service (enhanced GPRS), third generation partnership project (3GPP) long term evolution (LTE), third generation partnership project 2 (3GPP2) ultra-mobile broadband (UMB), high speed packet access (HSPA), Zigbee and other 802.XX wireless technologies and/or legacy telecommunication technologies, BLUETOOTH^{®}, Session Initiation Protocol (SIP), ZIGBEE^{®}, RF4CE protocol, WirelessHART protocol, 6LoWPAN (Ipv6 over Low power Wireless Area Networks), Z-Wave, an advanced and/or adaptive network technology (ANT), an ultra-wideband (UWB) standard protocol and/or other proprietary and/or non-proprietary communication protocols.

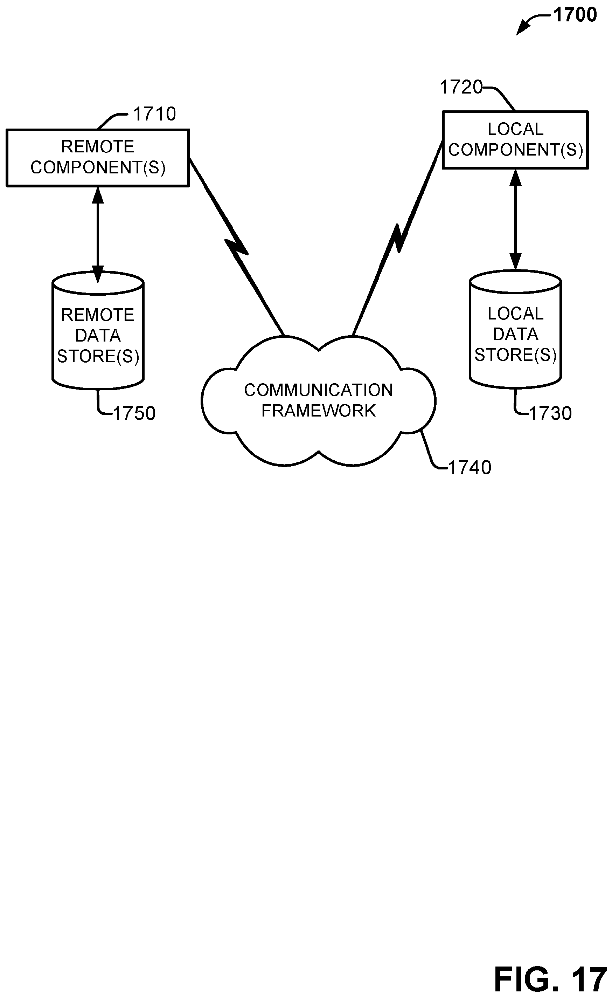

The molecular network generation system 602 can be associated with, such as accessible via, a cloud computing environment, such as the cloud computing environment 1700 of FIG. 17.

The molecular network generation system 602 can comprise a plurality of components. The components can comprise a memory 604, processor 606, bus 605, obtaining component 610, evaluating component 612, scoring component 614, updating 616, generating component 618, displaying component 620, parameterizing component 622, and/or executing component 624. Using these components, the molecular network generation system 602 can update the molecular network 640, generate a spectral data grouping 642 such as a MN visual 643 and/or output a query response 692, each in response to an unknown spectrum query 630.

Discussion next turns to the processor 606, memory 604 and bus 605 of the molecular network generation system 602. For example, in one or more embodiments, the molecular network generation system 602 can comprise the processor 606 (e.g., computer processing unit, microprocessor, classical processor, quantum processor and/or like processor). In one or more embodiments, a component associated with molecular network generation system 602, as described herein with or without reference to the one or more figures of the one or more embodiments, can comprise one or more computer and/or machine readable, writable and/or executable components and/or instructions that can be executed by processor 606 to provide performance of one or more processes defined by such component and/or instruction. In one or more embodiments, the processor 606 can comprise the obtaining component 610, evaluating component 612, scoring component 614, updating 616, generating component 618, displaying component 620, parameterizing component 622, and/or executing component 624.

In one or more embodiments, the molecular network generation system 602 can comprise the computer-readable memory 604 that can be operably connected to the processor 606. The memory 604 can store computer-executable instructions that, upon execution by the processor 606, can cause the processor 606 and/or one or more other components of the molecular network generation system 602 (e.g., obtaining component 610, evaluating component 612, scoring component 614, updating 616, generating component 618, displaying component 620, parameterizing component 622, and/or executing component 624) to perform one or more actions. In one or more embodiments, the memory 604 can store computer-executable components (e.g., obtaining component 610, evaluating component 612, scoring component 614, updating 616, generating component 618, displaying component 620, parameterizing component 622, and/or executing component 624).

The molecular network generation system 602 and/or a component thereof as described herein, can be communicatively, electrically, operatively, optically and/or otherwise coupled to one another via a bus 605. Bus 605 can comprise one or more of a memory bus, memory controller, peripheral bus, external bus, local bus, quantum bus and/or another type of bus that can employ one or more bus architectures. One or more of these examples of bus 605 can be employed.

In one or more embodiments, the molecular network generation system 602 can be coupled (e.g., communicatively, electrically, operatively, optically and/or like function) to one or more external systems (e.g., a non-illustrated electrical output production system, one or more output targets and/or an output target controller), sources and/or devices (e.g., classical and/or quantum computing devices, communication devices and/or like devices), such as via a network. In one or more embodiments, one or more of the components of the molecular network generation system 602 and/or of the non-limiting system 600 can reside in the cloud, and/or can reside locally in a local computing environment (e.g., at a specified location).

In addition to the processor 606 and/or memory 604 described above, the molecular network generation system 602 can comprise one or more computer and/or machine readable, writable and/or executable components and/or instructions that, when executed by processor 606, can provide performance of one or more operations defined by such component and/or instruction.

Discussion next turns to the additional components of the molecular network generation system 602 (e.g., obtaining component 610, evaluating component 612, scoring component 614, updating 616, generating component 618, displaying component 620, parameterizing component 622, and/or executing component 624), generally, the molecular network generation system 602 can perform a set of processes that can be separated into various steps comprising, but not limited to: unknown spectrum query analysis, updating of a molecular network 640, generation of a spectral data grouping 642, generation of a MN cloud visual 750 and/or operation of the MN 640 to obtain a query response 692.

First, it is noted that in one or more embodiments, the obtaining component 610, evaluating component 612, scoring component 614, updating 616, generating component 618, displaying component 620, parameterizing component 622, and/or executing component 624 can be implemented independently, without one or more other of the obtaining component 610, evaluating component 612, scoring component 614, updating 616, generating component 618, displaying component 620, parameterizing component 622, and/or executing component 624. Additionally and/or alternatively, the obtaining component 610, evaluating component 612, scoring component 614, updating 616, generating component 618, displaying component 620, parameterizing component 622, and/or executing component 624 can be comprised by a high-level analyzing component 603, one or more of the below-described functions of the obtaining component 610, evaluating component 612, scoring component 614, updating 616, generating component 618, displaying component 620, parameterizing component 622, and/or executing component 624 can be performed by the high-level analyzing component 603, and/or the obtaining component 610, evaluating component 612, scoring component 614, updating 616, generating component 618, displaying component 620, parameterizing component 622, and/or executing component 624 can be omitted with the high-level analyzing component 603 performing one or more of the below-described functions of the one or more omitted obtaining component 610, evaluating component 612, scoring component 614, updating 616, generating component 618, displaying component 620, parameterizing component 622, and/or executing component 624.

Turning first to the obtaining component 610, this component can generally acquire (e.g., obtain, locate, identify, request, download, etc.) a spectrum query 630 corresponding to an unknown spectrum 631 and/or to a known spectrum 634. In one or more cases, a spectrum query 630 can comprise unknown spectrum data 632, such as describing the unknown spectrum 631. In one or more embodiments, the obtaining component 610 can intercept, read and/or copy a query signal, communication, etc. intended for the molecular network 640 (e.g., where the MN 640 employs a processor, such as the processor 606 or another processor), such as comprising a spectrum query 630. For example, the spectrum query 630 can be in any suitable form, comprise data and/or metadata, comprise a spectrum or data underlying a spectrum, etc. For example, the spectrum query 630 can comprise unknown spectrum data 632. The unknown spectrum data 632 can comprise data defining at least a mass to charge ratios of ions exhibited at the unknown spectrum 631 and/or data underlying the spectrum.

Generally, the evaluating component 612 can execute a comparison of first spectrum data, comprising first mass to charge ratios of ions exhibited at a respective spectrum, to second spectrum data, comprising second mass to charge ratios of ions exhibited at a second respective spectrum. In one or more cases, this can comprises executing a comparison of first unknown spectrum data 632, comprising first mass to charge ratios of ions exhibited at a respective unknown spectrum 631, to second known spectrum data 638 , comprising second mass to charge ratios of ions exhibited at a second known analytical spectrum 634 of a library of known analytical spectra (e.g., a spectral library or library datastore 635). In other embodiments, unknown spectrum data 632 can be compared to unknown spectrum data 632, or known spectrum data 638 can be compared to known spectrum data 638.

That is, the evaluating component 612 can query and/or generate a command for access to the library datastore 635, can retrieve known spectra data 638 to any one or more, such as a plurality of, known analytical spectra 634. In one or more embodiments, the known analytical spectra 634 can be standard-based analytical spectra 634. In one or more embodiments, a known analytical spectrum 634 can previously have been comprised by a query related to an unknown spectrum. In one or more embodiments, a specified grouping of one or more known analytical spectra 634 can be employed, such as where specified by a user entity employing a user entity device communicatively couplable to the non-limiting system 600. For example, a user entity can have a prediction, guess, or hypothesis regarding a classification and/or other property of an compound underlying a spectrum related to the query 630, and thus the system (e.g., evaluating component 612) can base the specified grouping thereon.

Additionally, and/or alternatively, the evaluating component 612 can employ one or more other secondary properties 645 describing, defining and/or bounding an unknown spectrum 631 and/or known analytical spectrum 634 to execute the comparison. This can additionally and/or alternatively include types of ions, number of ions, ion intensity, activation energy, given energy level and/or reaction time, and need not include mass to charge ratios. This can additionally and/or alternatively include any one or more secondary properties 645 of the parameter classes illustrated at FIG. 9 (e.g., secondary property classes of general parameters 902, similarity score basis 904, multi-class or hierarchical classification 906, visualization 908, node ion visualization 910, to each be described in detail below).

In one or more cases, it is noted that a comparison executed by the evaluating component 612, and thus also by inherency the score executed by the scoring component 614 and the updating performed by the updating component 616, can be performed absent any additional comparison of the unknown spectrum 631 to any one or more other unknown spectra 631. In this way, the evaluating component 612, and thus the MN generation system 602, can provide for limited error (e.g., few to one data points of unknown data) being employed when updating a spectral library and generating a molecular network therefrom. In this way, by using known spectral data, and gradually building out the spectral data, unknown spectra can be classified and/or identified, while limiting compounded errors during the generating (e.g., as compared to existing frameworks that employ plural unknown data points when generating an update to a spectral library for an unknown compound).

Based at least on an initial comparison of a pair of spectra to one another by the evaluating component 612, the scoring component 614 can generally determine whether there is another spectrum (e.g., a known analytical spectrum 634) against which to compare a spectrum related to a spectrum query 630. This can be based on random determination of a quantity of analytical spectra 634 to analyze, on the particular known spectra 634 of a specified grouping, and/or on demand by control by a user entity.

Also based at least on an initial comparison of a pair of spectra to one another by the evaluating component 612, the scoring component 614 can generally generate a spectrum similarity score 704 (FIGS. 6 and 7) describing a level of similarity of the compared spectra to one another. Indeed, the scoring component 614 can perform such generation for each pairing of spectra data (e.g., unknown spectra data 632 and/or known spectra data 638) compared to one another by the evaluating component 612, based on each respective comparison output thereof. For example, a spectrum similarity score 704 can describe a similarity between the first mass to charge ratios of ions and the second mass to charge ratios of ions. For another example, the scoring component 614 can generate a spectrum similarity score 704 describing a comparison of an unknown spectrum data 632 to a known spectrum data 638.

In one or more embodiments, the scoring component 614 can employ a score algorithm, program, code and/or application such as a cosine, Tanimoto, Euclid, Dice, HighChem-HighRes, and/or National Institute of Standards and Technologies (NIST)-based algorithm.

For example, a spectrum similarity score 704 can be based on a scale of 0 to 1, with 0 meaning no similarity between the compared spectra and 1 meaning exact similarity between the compared spectra. Any fragmentation, subdivisions, etc. between 0 and 1 can be employed, e.g., any suitable number of decimal places.

Additionally, and/or alternatively, the evaluating component 612 can further associate a secondary property 645 of one spectrum data with another spectrum data, based on the comparison by the evaluating component 612 of the first spectrum data and the second spectrum data.

That is, the evaluating component 612 can identify identification metadata 646 (e.g., ID metadata 646) associated with a spectrum data 632, 638, which metadata 646 can define a secondary property 645. Such secondary property metadata 646 can be stored with the respective spectrum data 632, 638 and/or separate therefrom. In one or more embodiments, where an unknown spectrum data 632 is associated with a spectrum query 630, secondary property metadata 646 can be comprised by and/or separate from the unknown spectrum data 632.

This associating, as with the comparing of pairs of spectra data, and as with the generating of respective spectrum similarity scores, can be performed for a plurality of different spectra pairs (e.g., spectra data pairs), such as for each combination of a spectrum data associated with a spectrum query 630 and each spectrum data comprised by and/or employed by a molecular network 640 (e.g., comprised by a library datastore 635).

A secondary property 645 can be based on and/or comprise any one or more of the properties provided at FIG. 9, and/or any one or more properties provided above and/or below, without being limited thereto. For example, a secondary property 645 can be based on and/or comprise one or more physical properties, chemical properties, compound classes, chemical compound use class, substructural similarity, chemical structure descriptive class or superclass, toxicological characteristics, physico-chemical characteristics, metabolic pathway, enzymatic reactions, biological reactions, enzymes or catalysts, or organism or tissues, fragmentation kinetics, fragmentation kinetics breakdown curves, optimal energy, collision energies, chemical formulas, neutral losses, peak counts, commercial applications, domestic application and/or industrial applications. In one or more embodiments, any one or more such properties can be provided by the system as one that is associated with a parameter 906 (FIG. 9) for multi-class or hierarchical classification.

In one or more examples, a first secondary property 645 corresponding to first spectrum data 632, 638 can be associated by the evaluating component 612 with a second spectrum data 632, 638, based on a spectral similarity score 704 defining a similarity level between the first spectrum data and the second spectrum data. Additionally, and/or alternatively, a second secondary property 645 corresponding to second spectrum data 632, 638 can be associated by the evaluating component 612 with the first spectrum data 632, 638, based on a spectral similarity score 704 defining a similarity level between the first spectrum data and the second spectrum data.

For example, based on a spectrum similarity score satisfying (e.g., meeting and/or exceeding) a score threshold, the evaluating component 612 can determine to associate a secondary property 645 of one spectrum data of the respective pair of spectra data corresponding to the spectrum similarity score 704 also with the other spectrum data of the pair of spectra.

Additionally, and/or alternatively, in one or more examples, a first secondary property 645 corresponding to first spectrum data 632, 638 can be associated by the evaluating component 612 with a second spectrum data 632, 638, based on plurality of spectral similarity scores 704 defining respective similarity levels between the first spectrum data and a plurality of second spectra data (e.g., for a plurality of spectra, such as known analytical spectra 634, such as from a library datastore 635 employed by a molecular network 640). Additionally, and/or alternatively, a second secondary property 645 corresponding to second spectrum data 632, 638 can be associated by the evaluating component 612 with the first spectrum data 632, 638, based on plurality of spectral similarity scores 704 defining respective similarity levels between the first spectrum data and a plurality of second spectra data (e.g., for a plurality of spectra, such as known analytical spectra 634, such as from a library datastore 635 employed by a molecular network 640).

For example, based on a quantity of the plurality of spectrum similarity scores 704 satisfying (e.g., meeting and/or exceeding) a score threshold, based on an aggregation of the quantity of spectrum similarity scores 704 satisfying a score threshold, or based on an aggregation of all spectrum similarity scores 704 associated with the first spectrum data satisfying a score threshold, the evaluating component 612 can determine to associate a secondary property 645 of one spectrum data of the respective pair of spectra data corresponding to at least one spectrum similarity score 704 also with the other spectrum data of the pair of spectra. Additional associations can also be performed based thereon.

Additionally, and/or alternatively, in one or more examples, a first secondary property 645 corresponding to first spectrum data 632, 638 can be associated by the evaluating component 612 with a second spectrum data 632, 638, based on there being at least a specified quantity of spectra data (e.g., known or unknown, such as known analytical spectra 634, such as from a library datastore 635 employed by a molecular network 640), including the second spectrum data satisfying a comparison criteria. Additionally, and/or alternatively, a second secondary property 645 corresponding to second spectrum data 632, 638 can be associated by the evaluating component 612 with the first spectrum data 632, 638, based on there being at least a specified quantity of spectra data (e.g., known or unknown, such as known analytical spectra 634, such as from a library datastore 635 employed by a molecular network 640), including the second spectrum data satisfying a comparison criteria.

For example, at least a first quantity of second spectra data (e.g., a plurality of known analytical spectra data 638) can each have a second secondary property 645 associated therewith. The first quantity of second spectra data can be those having been compared to the first spectrum data resulting in a validated similarity by the evaluating component 612, such as having at least a specified similarity score 704 (e.g., satisfying a threshold each and/or in aggregate, such as based on an aggregation of the respective similarity scores 704). Based on the first quantity being reached and/or exceeded (e.g., the quantity being a threshold to be satisfied as described herein), the second secondary property can be associated with the first spectrum data, or the first secondary property can be associated with the second spectrum data, by the evaluating component 612.

Additionally, and/or alternatively, in one or more embodiments, a first secondary property 645 can only be associated with a second spectrum data where the first spectrum data has a high priority associated therewith (e.g., satisfying a priority threshold), such as based on respective identification metadata 646 corresponding thereto.

Additionally, and/or alternatively, in one or more embodiments, one or more secondary properties 645 can be associated prior to or instead of one or more other secondary properties 645. Such determination can be made by the system, such as based on historical data obtained from the data store 635 and/or by selection by a user entity using a computer device communicatively couplable to the non-limiting system 600. For example, a secondary property 645 of fragmentation kinetics can be associated first, before a secondary property 645 of collision energy. In a case where a first selection (e.g., the fragmentation kinetics) is not comprised by metadata corresponding to a spectrum data, a second selection can next take precedence (e.g., a collision energy).

Any two or more of the above-noted examples can be performed for a same first spectrum data 632, 638. Any two or more such associations can be performed at least partially in parallel with one another.

Turning next to the updating component 616, through use of the scoring component 614, and subsequent updating of the library datastore 635 underlying the MN 640, and thus by inherency updating the MN 640, poor relationship representation that is a deficiency of existing systems can be at least partially and/or fully remedied, thereby resulting in increased accuracy, efficiency and/or speed when processing a query 630 to the MN 640 or MN generation system 602.

That is, the updating component 616 generally can apply an update 642 to the spectral library (e.g., library datastore 635) based on the comparison of the first and second spectrum data to one another. In one or more embodiments, the updating component 616 can additionally and/or alternatively apply the update 642 to the MN 640 and/or direct the MN 640 to update based on the library datastore 635.

In one or more embodiments, the update 642 can comprise an unknown spectrum 631, unknown spectrum data 632, one or more spectrum similarity scores 704 generated, one or more secondary properties 645 associated and/or a temporary identifier for the unknown compound underlying the unknown spectrum data 632.

Turning next to the generating component 618, this component can generally generate a grouping of spectral data 642 (also referred to as a spectral data grouping 642) comprising the first spectrum data and the second spectrum data based on the spectrum similarity score 704 and on the associating of one or more secondary properties 645. A spectral data grouping 642 can comprise a list, matrix, log or any other grouping of data, metadata and/or labels defining a set of spectra data (e.g., any combination of known and/or unknown spectra data) that can be determined by the generating component 618 as being related, and thus having a relationship. The spectral data grouping 642 can be provided to a user entity (e.g., transmitted to and/or made available to a user entity computer device) in any suitable form, such as a list, matrix, log, etc. In one or more cases, the spectral data grouping 642 additionally and/or alternatively can be employed by the displaying component 620 to generate a molecular network visual 643, such as a cloud visual 700/750, as described below.

The relationship can be generally based on a combination of spectra similarity scores 704 and secondary properties 645 associated with the spectra data to be comprised by the spectral data grouping 642. In one or more examples, a spectral data grouping 642 can be based on a first specified range of spectra similarity scores 704 and on a second specified range of one or more secondary properties 645. In one or more cases, the second specified range can be based on the first specified range, or vice versa.

In one or more cases, selection of the first specified range and/or the second specified range can be based on a determination by the generating component 618 and/or on data associated with a spectrum query 630. Additionally, and/or alternatively, in one or more cases, selection of the first specified range can be based on a highest spectra similarity score 704 associated with the first spectrum data (e.g., which first spectrum data can correspond to a spectrum query 630). Additionally, and/or alternatively, in one or more cases, selection of the second specified range can be based on a secondary property 645 having been associated by the evaluating component 612 (either associated to the first spectrum data or to the second spectrum data.

Turning next to the illustration 700 of FIG. 7, and still referring to FIG. 6, illustrated is an example visualization of a portion of the MN 640, based on a single spectrum similarity score 704 generation, as can be generated by the generating component 618 and displaying component 620. For example, the generating component 618 can generate the data defining (e.g., underlying) the cloud visual illustration 700/750, and the displaying component 620 can generate the visual data and/or display the cloud visual illustration 700/750 at any suitable GUI, display, etc. communicatively couplable to the MN generation system 602 and/or non-limiting system 600 more generally.

The generating of the underlying data by the generating component 618 can employ know spectra data 638 of the library datastore 635 and the unknown spectrum data 632 for the unknown spectrum 631. The generating component 618 can generate a correspondence, tag, label, identifier, metadata, etc. corresponding to a relationship between the unknown spectrum 631 and the known analytical spectrum 634, where the relationship can correspond to the respective spectrum similarity score 704.

The display component 620, based on this generation, can generate visual data for generating a visualization of the MN 640 (e.g., or a portion thereof). This can include generating visualization data to represent the unknown spectrum 631 as a node 702U, the known analytical spectrum 634 as a node 702K, and the spectrum similarity score 704 as an edge 703 extending between the respective nodes 702. The edge 703 can comprise text next to, adjacent to, contiguous therewith, etc. that includes numbers of the spectrum similarity score 704, for easy visual reference by a user entity.

Turning to the illustration 750 of FIG. 7, illustrated is a MN cloud visual 750, comprising the illustration 700 and also comprising representation, in a cloud format, of a plurality of additional relationships between the unknown spectrum 631 (as the node 702U) and a plurality of additional known analytical spectra 634 as additional nodes 702.

Nodes 702 and/or edges 703 can be supplemented, by the generating component 618 (for generating the underlying data) and/or displaying component 620 (for visualizing the underlying data) with metadata, including compound classes, names, taxonomies, chemical families biochemical activity, and/or hydrophobicity, without being limited thereto, which can be reflected in a size, shape, color, fill color, fill pattern, border color, border thickness, length and/or positioning of a node 702 and/or edge 703.

As illustrated at the MN cloud visual 750, a first generation 711 of edges 703 can extend from the unknown node 702U to a first plurality of known nodes 702K. Also as illustrated at the MN cloud visual 750, a second generation 712, can extend from the first plurality of known nodes 702K to a second plurality of known nodes 702K. Indeed, any one or more generations of relationships can be visualized.

An identifier can be employed for one or more nodes 702 or edges 703. For example, a text identifier can be employed for nodes 702 based on an associated secondary property 645.

Further, such identifiers can be provided other than by text. For example, nodes 702 and/or edges 703 can be supplemented, by the generating component 618 (for generating the underlying data) and/or displaying component 620 (for visualizing the underlying data) with metadata, including compound classes, names, taxonomies, chemical families biochemical activity, and/or hydrophobicity, without being limited thereto, such as based on any secondary property 645 mentioned herein above and/or below, which can be reflected in a visual aspect, such as a size, shape, color, fill color, fill pattern, border color, border thickness, length and/or positioning of a node 702 and/or edge 703. For example, a first visual aspect can comprise a colored edge 703, and a second visual aspect can comprise a colored border of a node 702.

To provide the visual aspects, the displaying component 620 can evaluate the identification metadata 646 associated with the secondary properties 645 that are in turn associated with the spectrum data 632, 638 employed by a molecular network 640

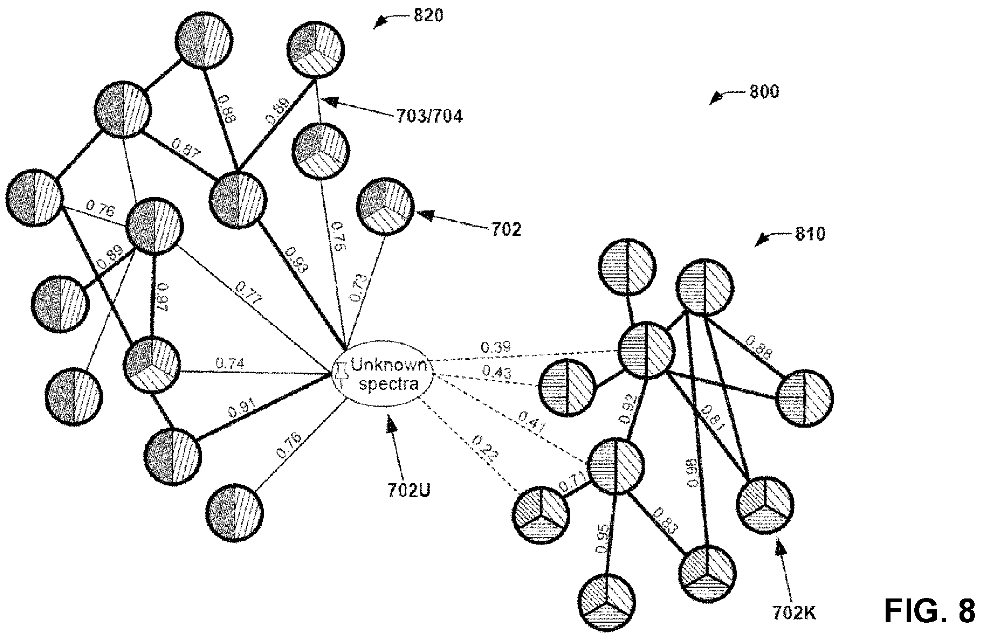

Turning briefly to the schematic diagram of FIG. 8, in one or more embodiments, the nodes 702 and/or edges 703 can be clickable, interactable with, interactive, etc., causing change in the library spectra visualized. For example, selecting a node 702 can cause that node 702 to become a center of a cloud visual 750 and/or to bring up a text box including correspondence information (e.g., chemical property, classification, relationship, etc.). For another example, selecting a node 702 can bring up a text box including definition of a property (e.g., color, thickness, fill, patterning etc.) of the node 702. For example, selecting an edge 703 can bring up a text box including reasoning or underlying calculation defining the spectrum similarity score 704 and/or other property (e.g., color, thickness, etc.) of the edge 703.

Also, as illustrated at FIG. 8, known nodes 702K can be visually separated into groups, such as spaced apart from one another, using different patterning, border color, etc. Such groups 810, 820 can be of any suitable number and can be based on any suitable parameter or parameters, as will be discussed below relative to FIG. 9.

Relative to either of FIG. 7 or FIG. 8, in one or more embodiments, selection of one or more subgroups of known analytical spectra 634 (and thus the corresponding nodes 702K) can be from an entire content of a spectral library, or portion of a spectral library, can consider collision-induced dissociation (CID), high energy C-trap dissociation (HCD), ultraviolet photodissociation (UVPD), or any other activation energy and given energy level or reaction time, and/or can be based on any other suitable correspondence (e.g., chemical family, classification, property and/or relationship). In one or more subgroups, known analytical spectra 634 can be employed from the given energy level. If known analytical spectra 634 at a given and/or specified property, e.g., activation energy, is not available, the nearest available energy level can be employed by the generating component 618. For example, if CID 20 is not available, CID 15, 25 or 30 can be used.

Also relative to either of FIG. 7 or FIG. 8, in one or more embodiments, simultaneous visualization of several nearest network families (e.g., plural MN cloud visuals 750) exhibiting spectral relationships relative to a query spectrum can be provided by the generating component 618 and the displaying component 620. This can allow for side-by-side visualization of different clouds, with or without an unknown spectrum node 702U being employed.

Turning now to FIG. 9, illustrated is a schematic diagram 900 of an interactive panel GUI that can be employed to edit one or more parameters 902, 904, 906, 908 and/or 910, without being limited thereto, employed by the one or more embodiments described herein to generate a molecular network visualization. Parameters that can be optimized can comprise, but are not limited to chemical taxonomies for compounds, color code on nodes, similarity score cut-off, compound representation such as name, formula (ticking dots), number of nodes and generations with type-in windows, and/or number of sharing ions with type-in windows.

For example, general parameters 902 can comprise number of connections to a node 702U, 702K or any node 702, number of generations to visualize/display, etc.

Similarity score basis parameters 904 can comprise selection of a basis on which the similarity scores 704 are based, e.g., Cosine, HighChem, NIST, etc.

Multi-class or hierarchical classification parameters 906 can comprise any suitable ranking or leveling of hierarchies, and/or any suitable set of multi-class classifications suitable for any number of ontologies, whether chemical, classical, biological, functional and/or toxicological. Two or more different such hierarchical classification parameter categories can be employed in one or more embodiments. For example, a set of multi-class chemical classifications can comprise, but is not limited to, drugs of abuse, natural compounds, surfactants, textile chemicals, extractables, leachables, marine toxins, person care products, cosmetic products, drugs, pesticides, etc.

Visualization parameters 908 can comprise edge thickness, edge color, edge length, node color, node patterning, node border thickness, node border color and/or node size.

Node visualization parameters 910 can comprise which different types of ions and/or numbers thereof, are to be comprised by any known analytical spectra 634 being employed as known nodes 702K. It is noted that use of any of these categories is non-limiting, and indeed, the categories themselves are non-limiting.

Any combination of the categories and/or parameters illustrated and/or additional non-illustrated categories and/or parameters can be employed by the generating component 618 and/or visualized at the interactive property customization GUI 900 by the displaying component 620.

In one or more embodiments, these parameters, as illustrated at the interactive property customization GUI 900, can be employed, modified, adjusted and/or applied by the parameterizing component 622, in combination with the generating component 618 and/or displaying component 620. For example, the parameterizing component 622 can apply a first property of a spectrum similarity score 704 as a first visual modification of the edge and can apply a second property of the known analytical spectrum 634 as a second visual modification of the respective node of the known analytical spectrum 634. For another example, the parameterizing component 622 can adjust at least one of the first visual modification or the second visual modification based on selection, at a graphical user interface comprising the visual, of a class of properties comprising properties other than at least one of the first property or the second property.

Put another way, operation of the parameterizing component 622 can allow for filtering of spectra data that are employed by the generating component 618 for generating the one or more spectral data groupings 642.

Turning now to the executing component 624, this component can generally generate a response 692 to the query 630, such as where the query 630 included an inquiry. Such inquiry can comprise, for example, determining a classification, relationship, chemical family, closest spectra, identification of, etc., without being limited thereto, of an unknown compound underlying the unknown spectrum 631. For example, the executing component 624 can identify a classification for the unknown spectrum 631 based on the visual comprising a set of visual elements corresponding to properties of the unknown spectrum 631 and the known analytical spectrum 634.

In summary, the one or more embodiments described herein can provide for comparison of unknown spectra 631 with a MN of highly curated spectral trees with different metadata taxonomies in once space (e.g., representing the known spectra 634), simultaneous visualization of several nearest network families (e.g., plural MN cloud visuals 750) exhibiting spectral relationships relative to a query spectrum, customizable visualization options (e.g., as illustrated at FIG. 9), and/or facilitation of a decision making process to accurately judge best hits (e.g., based on operation of the executing component 624).

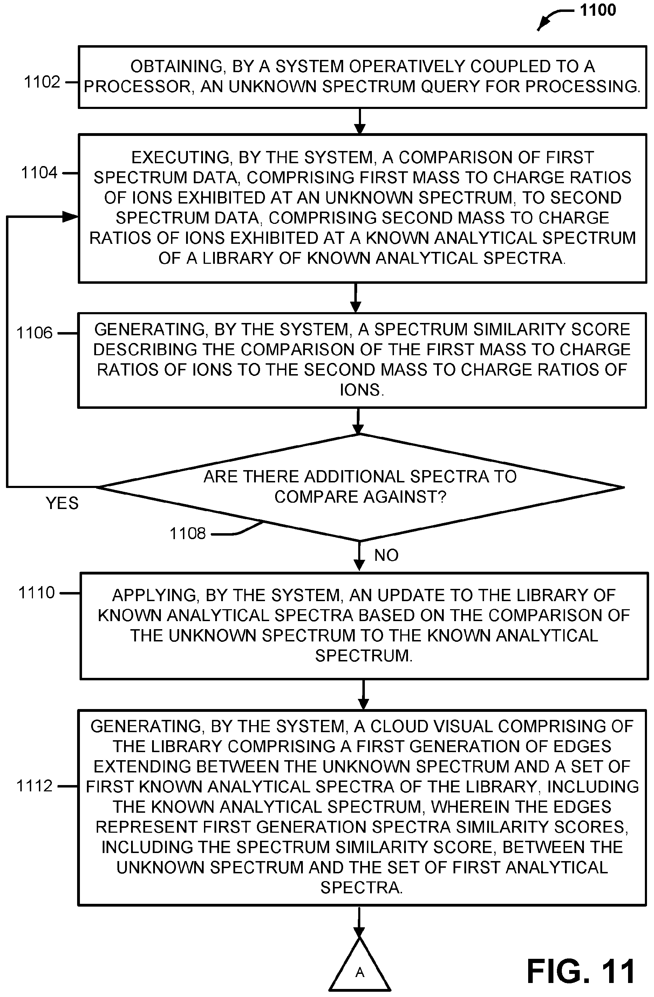

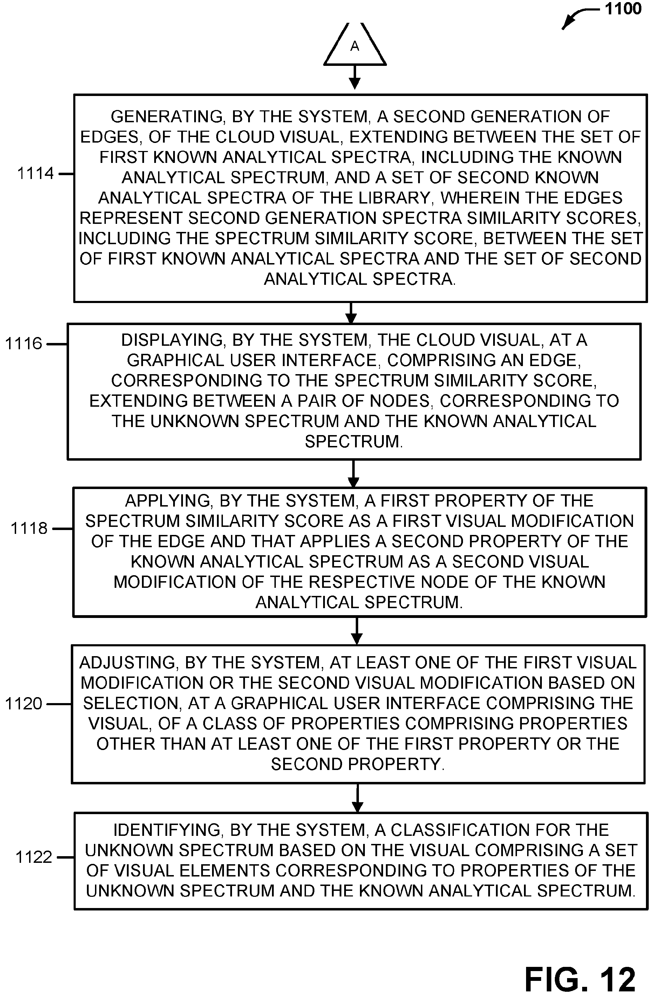

As a summary of the above-described components and/or functions thereof, referring next to FIGS. 11 and 12, illustrated is a flow diagram of an example, non-limiting method 1100 that can facilitate a process for molecular network generation, visualization and/or employment, in accordance with one or more embodiments described herein, such as the non-limiting system 600 of FIG. 6. While the non-limiting method 1100 is described relative to the non-limiting system 600 of FIG. 6, the non-limiting method 1100 can be applicable also to other systems described herein, such as the non-limiting system 500 of FIG. 5. Repetitive description of like elements and/or processes employed in respective embodiments is omitted for sake of brevity.

At 1102, the non-limiting method 1100 can comprise obtaining, by a system (e.g., obtaining component 610) an unknown spectrum query (e.g., spectrum query 630) for processing.

At 1104, the non-limiting method 1100 can comprise executing, by the system (e.g., evaluating component 612), a comparison of first spectrum data (e.g., unknown spectrum data 632), comprising first mass to charge ratios of ions exhibited at an unknown spectrum (e.g., unknown spectrum 631), to second spectrum data (e.g., known spectra data 638), comprising second mass to charge ratios of ions exhibited at a known analytical spectrum (e.g., known analytical spectra 634) of a library (e.g., library datastore 635) of known analytical spectra (e.g., known analytical spectra 634).

In one or more embodiments, the comparing is performed absent any additional comparison of the unknown spectrum to a second unknown spectrum.

At 1106, the non-limiting method 1100 can comprise generating, by the system (e.g., scoring component 614), a spectrum similarity score (e.g., spectrum similarity score 704) describing the comparison of the first mass to charge ratios of ions to the second mass to charge ratios of ions.

At 1108, the non-limiting method 1100 can comprise determining, by the system (e.g., scoring component 614), whether there are additional known spectra of the library against which to compare the unknown spectrum. If yes, the non-limiting method 1100 can proceed back to step 1104. If not, the non-limiting method can proceed forward to step 1110.

At 1110, the non-limiting method 1100 can comprise applying, by the system (e.g., updating component 616) an update to the library of known analytical spectra based on the comparison of the unknown spectrum to the known analytical spectrum.

At 1112, the non-limiting method 1100 can comprise generating, by the system (e.g., generating component 618), a cloud visual (e.g., MN cloud visual 750) comprising a first generation of edges (e.g., edges 703) extending between the unknown spectrum and a set of first known analytical spectra of the library, including the known analytical spectrum, wherein the edges represent first generation spectra similarity scores, including the spectrum similarity score, between the unknown spectrum and the set of first analytical spectra.

At 1114, the non-limiting method 1100 can comprise generating, by the system (e.g., generating component 618), a second generation of edges, of the cloud visual, extending between the set of first known analytical spectra, including the known analytical spectrum, and a set of second known analytical spectra of the library, wherein the edges represent second generation spectra similarity scores, including the spectrum similarity score, between the set of first known analytical spectra and the set of second analytical spectra.

At 1116, the non-limiting method 1100 can comprise displaying, by the system (e.g., displaying component 620), the cloud visual, at a graphical user interface (e.g., GUI 300), comprising an edge, corresponding to the spectrum similarity score, extending between a pair of nodes (e.g., nodes 702), corresponding to the unknown spectrum and the known analytical spectrum.

At 1118, the non-limiting method 1100 can comprise applying, by the system (e.g., parameterizing component 622), a first property (e.g., parameters 902-910) of the spectrum similarity score as a first visual modification (e.g., visual modification 690) of the edge and that applies a second property (e.g., parameters 902-910) of the known analytical spectrum as a second visual modification (e.g., visual modification 690) of the respective node of the known analytical spectrum.

At 1120, the non-limiting method 1100 can comprise adjusting, by the system (e.g., parameterizing component 622), at least one of the first visual modification or the second visual modification based on selection, at a graphical user interface comprising the visual, of a class of properties comprising properties other than at least one of the first property or the second property.

At 1122, the non-limiting method 1100 can comprise identifying, by the system (e.g., executing component 624), a classification (e.g., query response 692) for the unknown spectrum based on the visual comprising a set of visual elements corresponding to properties of the unknown spectrum and the known analytical spectrum.

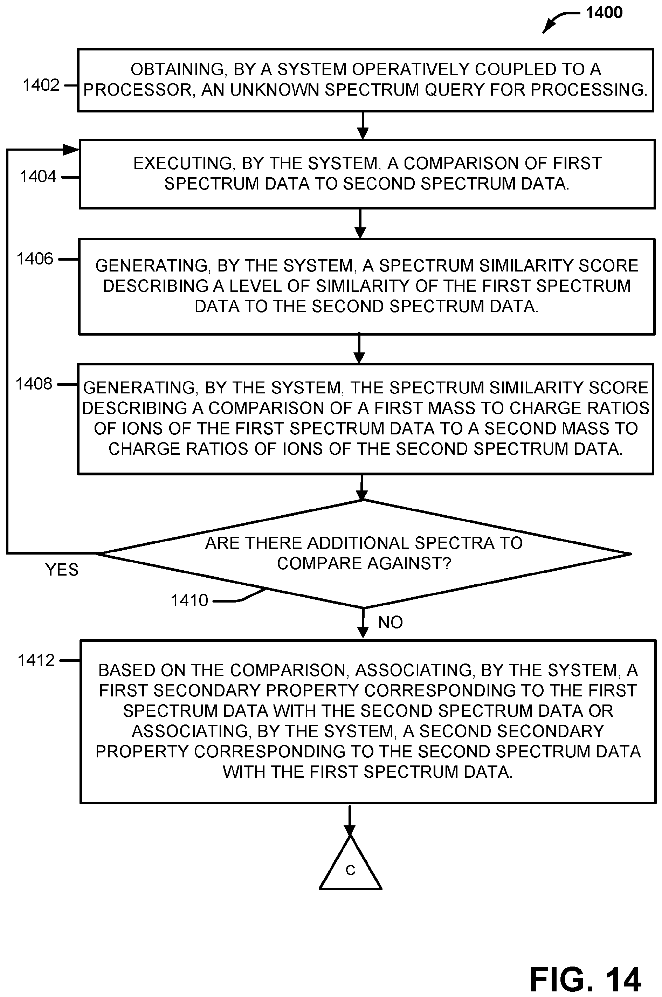

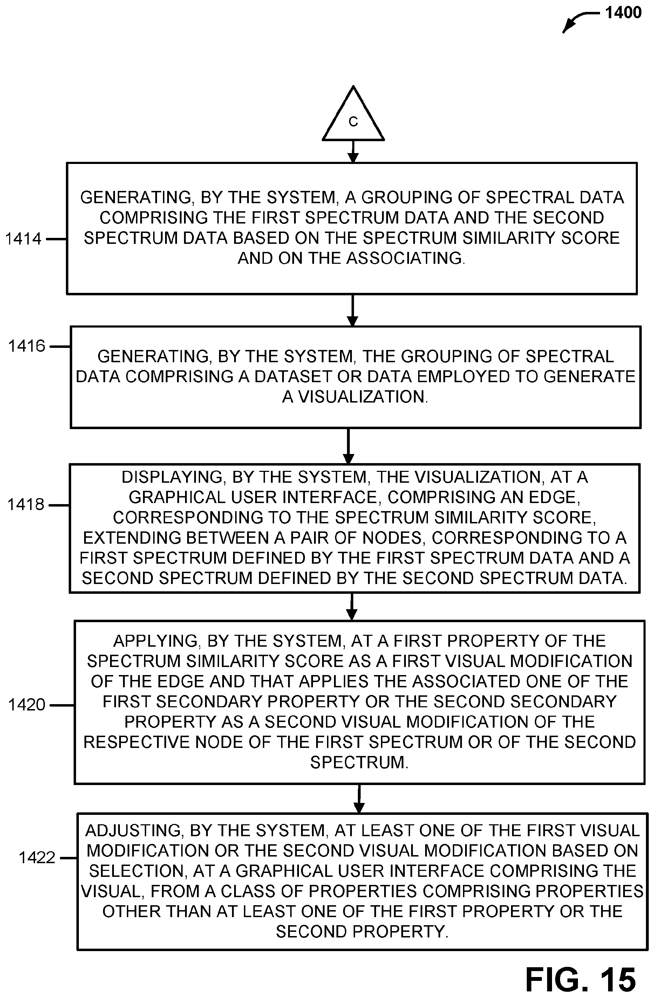

As another summary of the above-described components and/or functions thereof, referring next to FIGS. 14 and 15, illustrated is a flow diagram of an example, non-limiting method 1400 that can facilitate a process for molecular network generation, visualization and/or employment, in accordance with one or more embodiments described herein, such as the non-limiting system 600 of FIG. 6. While the non-limiting method 1400 is described relative to the non-limiting system 600 of FIG. 6, the non-limiting method 1400 can be applicable also to other systems described herein, such as the non-limiting system 500 of FIG. 5. Repetitive description of like elements and/or processes employed in respective embodiments is omitted for sake of brevity.

At 1402, the non-limiting method 1400 can comprise obtaining, by a system (e.g., obtaining component 610) an unknown spectrum query (e.g., spectrum query 630) for processing.

At 1404, the non-limiting method 1400 can comprise executing, by the system (e.g., evaluating component 612), a comparison of first spectrum data (e.g., spectrum data 632 or 638) to second spectrum data (e.g., spectrum data 632 or 638).

In one or more embodiments, the first spectrum data is an unknown spectrum data (e.g., unknown spectrum data 632) that is not comprised by a molecular network (e.g., molecular network 640) , and the second spectrum data is known spectrum data (e.g., known spectrum data 638) that is comprised by the molecular network.

At 1406, the non-limiting method 1400 can comprise generating, by the system (e.g., scoring component 614), a spectrum similarity score (e.g., spectrum similarity score 704) describing a level of similarity of the first spectrum data to the second spectrum data.

At 1408, the non-limiting method 1400 can comprise generating, by the system (e.g., scoring component 614), the spectrum similarity score describing a comparison of a first mass to charge ratios of ions of the first spectrum data to a second mass to charge ratios of ions of the second spectrum data.

At 1410, the non-limiting method 1400 can comprise determining, by the system (e.g., scoring component 614), whether there are additional known spectra of the library against which to compare the unknown spectrum. If yes, the non-limiting method 1400 can proceed back to step 1404. If not, the non-limiting method can proceed forward to step 1412.

At 1412, the non-limiting method 1400 can comprise based on the comparison, associating, by the system (e.g., evaluating component 612), a first secondary property (e.g., secondary property 645) corresponding to the first spectrum data with the second spectrum data or associating, by the system (e.g., evaluating component 612), a second secondary property (e.g., secondary property 645) corresponding to the second spectrum data with the first spectrum data.

In one or more embodiments, the associated one of the first secondary property or the second secondary property is defined by identification metadata (e.g., ID metadata 646) associated with the first spectrum data or the second spectrum data.

Additionally, and/or alternatively, in one or more embodiments, the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.

At 1414, the non-limiting method 1400 can comprise generating, by the system (e.g., generating component 618), a grouping of spectral data (e.g., spectral data grouping 642) comprising the first spectrum data and the second spectrum data based on the spectrum similarity score and on the associating.

At 1416, the non-limiting method 1400 can comprise generating, by the system (e.g., generating component 618), the grouping of spectral data comprising a dataset or data employed to generate a visualization (e.g., molecular network visual 643).

At 1418, the non-limiting method 1400 can comprise displaying, by the system (e.g., displaying component 620), the visualization, at a graphical user interface (e.g., GUI 300), comprising an edge (e.g., edge 703), corresponding to the spectrum similarity score, extending between a pair of nodes (e.g., nodes 702), corresponding to a first spectrum (e.g., spectrum 631 or 634) defined by the first spectrum data and a second spectrum (e.g., spectrum 631 or 634) defined by the second spectrum data.

At 1420, the non-limiting method 1400 can comprise applying, by the system (e.g., parameterizing component 622), a first property (e.g., parameters 902-910) of the spectrum similarity score as a first visual modification (e.g., visual modification 690) of the edge and that applies the associated one of the first secondary property or the second secondary property as a second visual modification (e.g., visual modification 690) of the respective node of the first spectrum or of the second spectrum.

At 1422, the non-limiting method 1400 can comprise adjusting, by the system (e.g., parameterizing component 622), at least one of the first visual modification or the second visual modification based on selection, at a graphical user interface comprising the visual, from a class of properties comprising properties other than at least one of the first property or the second property.

### Additional Summary

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. In addition, the computer-implemented and non-computer-implemented methodologies could alternatively be represented as a series of interrelated states via a state diagram or events. Additionally, the computer-implemented methodologies described hereinafter and throughout this specification are capable of being stored on an article of manufacture for transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In summary, one or more systems, computer program products and/or computer-implemented methods provided herein relate to a process for molecular network generation. A system can comprise a memory 504, 604 that stores, and a processor 506, 606 that executes, computer executable components. The computer executable components can comprise an evaluating component 512, 612 that executes a comparison of first spectrum data 532, 632, comprising first mass to charge ratios of ions exhibited at an unknown spectrum 531, 631, to second spectrum data 538, 638 , comprising second mass to charge ratios of ions exhibited at a known analytical spectrum 534, 634 of a library 535, 635 of known analytical spectra, and an updating component 516, 616 that applies an update 542, 642 to the library 535, 635 of known analytical spectra based on the comparison of the unknown spectrum 531, 631 to the known analytical spectrum 534, 634.

In another summary, one or more systems, computer program products and/or computer-implemented methods provided herein relate to a process for molecular network use. A system can comprise a memory 504, 604 that stores, and a processor 506, 606 that executes, computer executable components. The computer executable components can comprise an evaluating component 512, 612 that executes a comparison of first spectrum data 532, 632 to second spectrum data 532, 632, a scoring component 514, 614 that, based on the comparison, generates a spectrum similarity score 544, 704 describing a level of similarity of the first spectrum data 532, 632 to the second spectrum data 532, 632, a parameterizing component 522, 622 that, based on the comparison, associates a first secondary property 545, 645 corresponding to the first spectrum data 532, 632 with the second spectrum data 532, 632 or associates a second secondary property 545, 645 corresponding to the second spectrum data 532, 632 with the first spectrum data 532, 632, and a generating component 518, 618 that generates a grouping of spectral data 542, 642 comprising the first spectrum data 532, 632 and the second spectrum data 532, 632 based on the spectrum similarity score 544, 704 and on the associating.

The one or more embodiments described herein can employ a novel system that provides for limited error (e.g., few to one data points of unknown data) being employed when updating a spectral library and generating a molecular network therefrom. In this way, by using known spectral data, and gradually building out the spectral data, unknown spectra can be classified and/or identified, while limiting compounded errors during the generating (e.g., as compared to existing frameworks that employ plural unknown data points when generating an update to a spectral library for an unknown compound).

Additionally, and/or alternatively, the one or more embodiments described herein can employ the novel system to provide greater accuracy and/or more specific spectral data groupings to further limit unusable spectral data that is returned based on a query and/or based on one or more parameter adjustments and/or filtering adjustments performed by and/or requested by a user entity. For example, in one or more embodiments described herein, a spectral data grouping can be generated from a molecular network and provided as any one or more of a visual, data, metadata, etc. The spectral data grouping can be generated based on one or more of a) one or more similarity scores between pairs of spectrum data or b) one or more secondary properties of at least one of the spectral data of the pairs of spectrum data. That is, in one or more embodiments, a spectral data grouping can be based on similarity scores and on a secondary property. In one or more other embodiments, a spectral data grouping can be based on a first secondary property and at least one other secondary property.

The one or more embodiments described herein can be employed to generate a molecular network that can provide varying outputs during use of the molecular network. For example, based on visual aspects of a format of a MN cloud, such as coloring, line thicknesses, shapes and/or distances between different aspects of the MN cloud, a user entity, or the system itself, can predict one or more chemical properties and/or relationships corresponding to an unknown spectra. These one or more chemical properties and/or relationships can comprise chemical class, chemical use, similar compounds, etc.

The one or more embodiments described herein can provide the molecular network visual being a dynamically adjustable visual that can provide varied visualization types and/or customization of visualized chemical relationships and/or properties. For example, dynamic adjustability can be found in functioning of the generated molecular network (MN), where a user entity can interact with the visual display to vary chemical classes, chemical properties, sizes and/or distances of varying MN aspects, etc. Varied visualizations can comprise large MN clouds, customized clouds based on one or more specified parameters, plural clouds displayed at a same time as one another, etc. Customization can be provided by use of a graphical user interface (GUI) allowing for different chemical properties and/or relationships to be represented by nodes, edges, borders of nodes and/or edges, fill of nodes and/or edges, thickness of lines within a cloud, distances between nodes, etc.

The one or more embodiments described herein can be implemented within, in connection with and/or coupled to a scientific imaging device.

The one or more embodiments disclosed herein can be applied on a plug-and-play basis to various architectures of existing spectral library and/or library datastores of spectral data. That is, the one or more embodiments described herein can generate a molecular network comprising a visual representing a plurality of chemical relationships regardless of data structure of a spectral library.

Indeed, in view of the one or more embodiments described herein, a practical application of the one or more systems, computer-implemented methods and/or computer program products described herein can be ability to provide grouping of spectral data based on a combination of secondary properties corresponding to spectral data and/or on a combination of at least one secondary property and one or more similarity scores corresponding to spectral data. This spectral data grouping can be more narrow, specific and/or accurate, based on such combinations, than can be provided by existing frameworks. Relative to the spectral data grouping, a molecular network visual can be realized and displayed. The spectral data grouping, in data, metadata and/or a visual, can allow for an understanding of a chemical property, relationship and/or classification of and/or corresponding to the unknown spectrum query. That is, as compared to existing frameworks that cannot provide this ability, the one or more embodiments described herein can provide a new result that was previously unavailable, e.g., an accurate spectral data grouping and/or MN updating. In one or more cases, this can be performed absent use of a plurality of unknown data points which can undesirably compound errors related to the MN generating and/or updating.

These are useful and practical applications of computers, thus providing enhanced (e.g., improved and/or optimized) material analysis and image modification output. Overall, such computerized tools can constitute a concrete and tangible technical improvement in the fields of material analysis, and more particularly in material analysis using molecular networks, spectral data groupings, and/or molecular network cloud visuals generated therefrom.

Furthermore, one or more embodiments described herein can be employed in a real-world system based on the disclosed teachings. For example, the one or more embodiments described herein can provide the spectral data grouping, generated based on one or more similarity scores and based upon one or more associated secondary properties that have been associated based on the one or more similarity scores, in a data, metadata and/or visualized (e.g., graphic-based) form. Additionally, and/or alternatively, this process can be employed to generate at least a portion of a molecular network by updating the molecular network (e.g., by updating the spectral library underlying the molecular network) based at least on the associating of the one or more secondary properties. These can be useful processes for varying industries employing material analysis, product manufacturing, quality control and/or the like. The embodiments disclosed herein thus can provide improvements to scientific instrument technology (e.g., improvements in the computer technology supporting such scientific instruments, among other improvements).

In one or more cases, based thereon, one or more molecular network cloud visuals (and data underlying the cloud visuals) can be generated and analyzed, thereby resulting in a determination of one or more chemical correspondences (e.g., chemical properties, relationships and/or classification) for the one or more unknown compound queries. These likewise can be useful processes for varying industries employing material analysis, product manufacturing, quality control and/or the like.

Moreover, the one or more embodiments described herein can achieve a level of scale of operation. For example, two or more compound queries can be analyzed and two or more corresponding spectral libraries updated based thereon, at least partially in parallel with one another, while applying separate processes for one spectrum query as compared to another spectrum query. In one or more cases, any combination of two or more spectral data groupings and/or MN cloud visuals can be generated at least partially at a same time as one another.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

One or more embodiments described herein can be, in one or more embodiments, inherently and/or inextricably tied to computer technology and cannot be implemented outside of a computing environment. For example, one or more processes performed by one or more embodiments described herein can more efficiently, and even more feasibly, provide program and/or program instruction execution, such as relative to material analysis using molecular network generation and/or visualization, as compared to existing systems and/or techniques using molecular network generation and/or visualization. Systems, computer-implemented methods and/or computer program products providing performance of these processes are of great utility in the fields of material analysis, such for determining one or more chemical correspondences (e.g., chemical properties, relationships and/or classification) for one or more unknown compound queries and cannot be equally practicably implemented in a sensible way outside of a computing environment.

One or more embodiments described herein can employ hardware and/or software to solve problems that are highly technical, that are not abstract, and that cannot be performed as a set of mental acts by a human. For example, a human, or even thousands of humans, cannot efficiently, accurately and/or effectively analyze computer data/metadata defining spectra for a plurality of compounds, and/or generate a digital display visual of a molecular network based on a plurality of spectral data, while employing a plurality of different chemical correspondences to bound and/or adjust the display visual as the one or more embodiments described herein can provide this process. Moreover, neither can the human mind nor a human with pen and paper conduct one or more of these processes, as conducted by one or more embodiments described herein.

In one or more embodiments, one or more of the processes described herein can be performed by one or more specialized computers (e.g., a specialized processing unit, a specialized classical computer, a specialized quantum computer, a specialized hybrid classical/quantum system and/or another type of specialized computer) to execute defined tasks related to the one or more technologies describe above. One or more embodiments described herein and/or components thereof can be employed to solve new problems that arise through advancements in technologies mentioned above, employment of quantum computing systems, cloud computing systems, computer architecture and/or another technology.

One or more embodiments described herein can be fully operational towards performing one or more other functions (e.g., fully powered on, fully executed and/or another function) while also performing one or more of the one or more operations described herein.

To provide additional summary, a listing of embodiments and features thereof is next provided.

A system, comprising: a memory that stores computer executable components; and a processor that executes the computer executable components stored in the memory, wherein the computer executable components comprise: an evaluating component that executes a comparison of first spectrum data, comprising first mass to charge ratios of ions exhibited at an unknown spectrum, to second spectrum data, comprising second mass to charge ratios of ions exhibited at a known analytical spectrum of a library of known analytical spectra; and an updating component that applies an update to the library of known analytical spectra based on the comparison of the unknown spectrum to the known analytical spectrum.

The system of the preceding paragraph, wherein the update is applied absent any additional comparison of the unknown spectrum to a second unknown spectrum.

The system of any preceding paragraph, wherein the computer executable components further comprise: a scoring component that generates a spectrum similarity score describing the comparison of the first mass to charge ratios of ions to the second mass to charge ratios of ions.

The system of any preceding paragraph, wherein the computer executable components further comprise: a displaying component that displays a visual, at a graphical user interface, comprising an edge, corresponding to the spectrum similarity score, extending between a pair of nodes, corresponding to the unknown spectrum and the known analytical spectrum.

The system of any preceding paragraph, wherein the computer executable components further comprise: a generating component that generates a cloud visual comprising a first generation of edges extending between the unknown spectrum and a set of first known analytical spectra of the library, including the known analytical spectrum, wherein the edges represent first generation spectra similarity scores, including the spectrum similarity score, between the unknown spectrum and the set of first analytical spectra.

The system of any preceding paragraph, wherein the generating component further generates a second generation of edges, of the cloud visual, extending between the set of first known analytical spectra, including the known analytical spectrum, and a set of second known analytical spectra of the library, wherein the edges represent second generation spectra similarity scores, including the spectrum similarity score, between the set of first known analytical spectra and the set of second analytical spectra.

The system of any preceding paragraph, wherein the computer executable components further comprise: a displaying component that displays a visual comprising the spectrum similarity score illustrated as an edge between the unknown spectrum and the known analytical spectrum illustrated as a pair of nodes; and a parameterizing component that applies a first property of the spectrum similarity score as a first visual modification of the edge and that applies a second property of the known analytical spectrum as a second visual modification of the respective node of the known analytical spectrum.

The system of any preceding paragraph, wherein the parameterizing component adjusts at least one of the first visual modification or the second visual modification based on selection, at a graphical user interface comprising the visual, of a class of properties comprising properties other than at least one of the first property or the second property.

The system of any preceding paragraph, wherein the computer executable components further comprise: a displaying component that displays a visual comprising the spectrum similarity score illustrated as an edge between the unknown spectrum and the known analytical spectrum illustrated as a pair of nodes; and an executing component that identifies a classification for the unknown spectrum based on the visual comprising a set of visual elements corresponding to properties of the unknown spectrum and the known analytical spectrum.

A computer-implemented method, comprising: executing, by a system operatively coupled to a processor, a comparison of first spectrum data, comprising first mass to charge ratios of ions exhibited at an unknown spectrum, to second spectrum data, comprising second mass to charge ratios of ions exhibited at a known analytical spectrum of a library of known analytical spectra; and applying, by the system, an update to the library of known analytical spectra based on the comparison of the unknown spectrum to the known analytical spectrum.

The computer-implemented method of the preceding paragraph, further comprising: applying, by the system, the update absent any additional comparison of the unknown spectrum to a second unknown spectrum.

The computer-implemented method of any preceding paragraph, further comprising: generating, by the system, a spectrum similarity score describing the comparison of the first mass to charge ratios of ions to the second mass to charge ratios of ions.

The computer-implemented method of any preceding paragraph, further comprising: displaying, by the system, a visual, at a graphical user interface, comprising an edge, corresponding to the spectrum similarity score, extending between a pair of nodes, corresponding to the unknown spectrum and the known analytical spectrum.

The computer-implemented method of any preceding paragraph, further comprising: generating, by the system, a cloud visual comprising a first generation of edges extending between the unknown spectrum and a set of first known analytical spectra of the library, including the known analytical spectrum, wherein the edges represent first generation spectra similarity scores, including the spectrum similarity score, between the unknown spectrum and the set of first analytical spectra; and generating, by the system, a second generation of edges, of the cloud visual, extending between the set of first known analytical spectra, including the known analytical spectrum, and a set of second known analytical spectra of the library, wherein the edges represent second generation spectra similarity scores, including the spectrum similarity score, between the set of first known analytical spectra and the set of second analytical spectra.

The computer-implemented method of any preceding paragraph, further comprising: displaying, by the system, a visual comprising the spectrum similarity score illustrated as an edge between the unknown spectrum and the known analytical spectrum illustrated as a pair of nodes; and identifying, by the system, a classification for the unknown spectrum based on the visual comprising a set of visual elements corresponding to properties of the unknown spectrum and the known analytical spectrum.

A computer program product facilitating a process for updating a library of known known analytical spectra with an unknown spectrum, the computer program product comprising a computer readable storage medium having program instructions embodied therewith, and the program instructions executable by a processor to cause the processor to: execute, by the processor, a comparison of first spectrum data, comprising first mass to charge ratios of ions exhibited at an unknown spectrum, to second spectrum data, comprising second mass to charge ratios of ions exhibited at a known analytical spectrum of a library of known analytical spectra; and apply, by the processor, an update to the library of known analytical spectra based on the comparison of the unknown spectrum to the known analytical spectrum.

The computer program product of the preceding paragraph, wherein the program instructions are further executable by the processor to cause the processor to: apply, by the processor, the update absent any additional comparison of the unknown spectrum to a second unknown spectrum.

The computer program product of any preceding paragraph, wherein the program instructions are further executable by the processor to cause the processor to: generate, by the processor, a spectrum similarity score describing the comparison of the first mass to charge ratios of ions to the second mass to charge ratios of ions.

The computer program product of any preceding paragraph, wherein the program instructions are further executable by the processor to cause the processor to: display, by the processor, a visual, at a graphical user interface, comprising an edge, corresponding to the spectrum similarity score, extending between a pair of nodes, corresponding to the unknown spectrum and the known analytical spectrum.

The computer program product of any preceding paragraph, wherein the program instructions are further executable by the processor to cause the processor to: display, by the processor, a visual comprising the spectrum similarity score illustrated as an edge between the unknown spectrum and the known analytical spectrum illustrated as a pair of nodes; and identify, by the processor, a classification for the unknown spectrum based on the visual comprising a set of visual elements corresponding to properties of the unknown spectrum and the known analytical spectrum.

A system, comprising: a memory that stores computer executable components; and a processor that executes the computer executable components stored in the memory, wherein the computer executable components comprise: an evaluating component that executes a comparison of first spectrum data to second spectrum data; a scoring component that, based on the comparison, generates a spectrum similarity score describing a level of similarity of the first spectrum data to the second spectrum data; a parameterizing component that, based on the comparison, associates a first secondary property corresponding to the first spectrum data with the second spectrum data or associates a second secondary property corresponding to the second spectrum data with the first spectrum data; and a generating component that generates a grouping of spectral data comprising the first spectrum data and the second spectrum data based on the spectrum similarity score and on the associating.

The system of the preceding paragraph, wherein the grouping of spectral data comprises a dataset or data employed to generate a visualization.

The system of any preceding paragraph, wherein the associated one of the first secondary property or the second secondary property is defined by identification metadata associated with the first spectrum data or the second spectrum data.

The system of any preceding paragraph, wherein the scoring component generates the spectrum similarity score describing a comparison of a first mass to charge ratios of ions of the first spectrum data to a second mass to charge ratios of ions of the second spectrum data.

The system of any preceding paragraph, wherein the first spectrum data is an unknown spectrum data that is not comprised by a molecular network, and wherein the second spectrum data is known spectrum data that is comprised by the molecular network.

The system of any preceding paragraph, wherein the first spectrum data is a first unknown spectrum data, and wherein the second spectrum data is a second unknown spectrum data.

The system of any preceding paragraph, wherein the associated one of the first secondary property or the second secondary property comprises one or more fragmentation kinetics, collision energy, neutral losses or peak counts.

The system of any preceding paragraph, wherein the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.

The system of any preceding paragraph, wherein the computer executable components further comprise: a displaying component that displays a visual, at a graphical user interface, comprising an edge, corresponding to the spectrum similarity score, extending between a pair of nodes, corresponding to a first spectrum defined by the first spectrum data and a second spectrum defined by the second spectrum data.

The system of any preceding paragraph, wherein the computer executable components further comprise: a parameterizing component that applies a first property of the spectrum similarity score as a first visual modification of the edge and that applies the associated one of the first secondary property or the second secondary property as a second visual modification of the respective node of the first spectrum or of the second spectrum.

The system of any preceding paragraph, wherein the parameterizing component adjusts at least one of the first visual modification or the second visual modification based on selection, at a graphical user interface comprising the visual, from a class of properties comprising properties other than at least one of the first property or the second property.

A computer-implemented method, comprising: executing, by a system operatively coupled to a processor, a comparison of first spectrum data to second spectrum data; based on the comparison, generating, by the system, a spectrum similarity score describing a level of similarity of the first spectrum data to the second spectrum data; based on the comparison, associating, by the system, a first secondary property corresponding to the first spectrum data with the second spectrum data or associating, by the system, a second secondary property corresponding to the second spectrum data with the first spectrum data; and generating, by the system, a grouping of spectral data comprising the first spectrum data and the second spectrum data based on the spectrum similarity score and on the associating.

The computer-implemented method of the preceding paragraph, wherein the grouping of spectral data comprises a dataset or data employed to generate a visualization.

The computer-implemented method of any preceding paragraph, wherein the associated one of the first secondary property or the second secondary property is defined by identification metadata associated with the first spectrum data or the second spectrum data.

The computer-implemented method of any preceding paragraph, further comprising: generating, by the system, the spectrum similarity score describing a comparison of a first mass to charge ratios of ions of the first spectrum data to a second mass to charge ratios of ions of the second spectrum data.

The computer-implemented method of any preceding paragraph, wherein the first spectrum data is an unknown spectrum data that is not comprised by a molecular network, and wherein the second spectrum data is known spectrum data that is comprised by the molecular network.

The computer-implemented method of any preceding paragraph, wherein the first spectrum data is a first unknown spectrum data, and wherein the second spectrum data is a second unknown spectrum data.

The computer-implemented method of any preceding paragraph, wherein the associated one of the first secondary property or the second secondary property comprises one or more fragmentation kinetics, collision energy, neutral losses or peak counts.

The computer-implemented method of any preceding paragraph, wherein the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.

A computer program product facilitating a process for generation of one or more spectral data groupings, the computer program product comprising a computer readable storage medium having program instructions embodied therewith, and the program instructions executable by a processor to cause the processor to: execute, by the processor, a comparison of first spectrum data to second spectrum data; based on the comparison, generate, by the processor, a spectrum similarity score describing a level of similarity of the first spectrum data to the second spectrum data; based on the comparison, associate, by the processor, a first secondary property corresponding to the first spectrum data with the second spectrum data or associate, by the processor, a second secondary property corresponding to the second spectrum data with the first spectrum data; and generate, by the processor, a grouping of spectral data comprising the first spectrum data and the second spectrum data based on the spectrum similarity score and on the associating.

The computer program product of the preceding paragraph, wherein the grouping of spectral data comprises a dataset or data employed to generate a visualization.

The computer program product of any preceding paragraph, wherein the associated one of the first secondary property or the second secondary property is defined by identification metadata associated with the first spectrum data or the second spectrum data.

The computer program product of any preceding paragraph, wherein the first spectrum data is an unknown spectrum data that is not comprised by a molecular network, and wherein the second spectrum data is known spectrum data that is comprised by the molecular network.

The computer program product of any preceding paragraph, wherein the first spectrum data is a first unknown spectrum data, and wherein the second spectrum data is a second unknown spectrum data.

The computer program product of any preceding paragraph, wherein the associated one of the first secondary property or the second secondary property comprises one or more fragmentation kinetics, collision energy, neutral losses or peak counts.

The computer program product of any preceding paragraph, wherein the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.

### Scientific Instrument System Description

Turning next to FIG. 16, a detailed description is provided of additional context for the one or more embodiments described herein at FIGS. 1-15. One or more computing devices implementing any of the scientific instrument modules or methods disclosed herein can be part of a scientific instrument system. FIG. 16 illustrates a block diagram of an example scientific instrument system 1600 in which one or more of the scientific instrument methods or other methods disclosed herein can be performed, in accordance with various embodiments described herein. The scientific instrument modules and methods disclosed herein (e.g., the scientific instrument module 100 of FIG. 1 and the method 200 of FIG. 2) can be implemented by one or more of the scientific instrument 1610, the user local computing device 1620, the service local computing device 1630, and/or the remote computing device 1640 of the scientific instrument system 1600.

Any of the scientific instrument 1610, the user local computing device 1620, the service local computing device 1630, and/or the remote computing device 1640 can include any of the embodiments of the computing device 400 discussed herein with reference to FIG. 4, and any of the scientific instrument 1610, the user local computing device 1620, the service local computing device 1630, and/or the remote computing device 1640 can take the form of any appropriate one or more of the embodiments of the computing device 400 discussed herein with reference to FIG. 4.

One or more of the scientific instrument 1610, the user local computing device 1620, the service local computing device 1630, and/or the remote computing device 1640 can include a processing device 1602, a storage device 1604, and/or an interface device 1606. The processing device 1602 can take any suitable form, including the form of any of the processors 402 discussed herein with reference to FIG. 4. The processing devices 1602 included in different ones of the scientific instrument 1610, the user local computing device 1620, the service local computing device 1630, and/or the remote computing device 1640 can take the same form or different forms. The storage device 1604 can take any suitable form, including the form of any of the storage devices 404 discussed herein with reference to FIG. 4. The storage devices 1604 included in different ones of the scientific instrument 1610, the user local computing device 1620, the service local computing device 1630, and/or the remote computing device 1640 can take the same form or different forms. The interface device 1606 can take any suitable form, including the form of any of the interface devices 406 discussed herein with reference to FIG. 4. The interface devices 1606 included in different ones of the scientific instrument 1610, the user local computing device 1620, the service local computing device 1630, and/or the remote computing device 1640 can take the same form or different forms.

The scientific instrument 1610, the user local computing device 1620, the service local computing device 1630, and/or the remote computing device 1640 can be in communication with other elements of the scientific instrument system 1600 via communication pathways 1608. The communication pathways 1608 can communicatively couple the interface devices 1606 of different ones of the elements of the scientific instrument system 1600, as shown, and can be wired or wireless communication pathways (e.g., in accordance with any of the communication techniques discussed herein with reference to the interface devices 406 of the computing device 400 of FIG. 4). The particular scientific instrument system 1600 depicted in FIG. 16 includes communication pathways between each pair of the scientific instrument 1610, the user local computing device 1620, the service local computing device 1630, and the remote computing device 1640, but this "fully connected" implementation is simply illustrative, and in various embodiments, various ones of the communication pathways 1608 can be omitted. For example, in one or more embodiments, a service local computing device 1630 can omit a direct communication pathway 1608 between its interface device 1606 and the interface device 1606 of the scientific instrument 1610, but can instead communicate with the scientific instrument 1610 via the communication pathway 1608 between the service local computing device 1630 and the user local computing device 1620 and/or the communication pathway 1608 between the user local computing device 1620 and the scientific instrument 1610.

The scientific instrument 1610 can include any appropriate scientific instrument, such as a separation or MS instrument, or other instrument facilitating material analysis.

The user local computing device 1620 can be a computing device (e.g., in accordance with any of the embodiments of the computing device 400 discussed herein) that is local to a user of the scientific instrument 1610. In one or more embodiments, the user local computing device 1620 can also be local to the scientific instrument 1610, but this need not be the case; for example, a user local computing device 1620 that is associated with a home, office or other building associated with a user entity can be remote from, but in communication with, the scientific instrument 1610 so that the user entity can use the user local computing device 1620 to control and/or access data from the scientific instrument 1610. In one or more embodiments, the user local computing device 1620 can be a laptop, smartphone, or tablet device. In one or more embodiments the user local computing device 1620 can be a portable computing device. In one or more embodiments, the user local computing device 1620 can deployed in the field.

The service local computing device 1630 can be a computing device (e.g., in accordance with any of the embodiments of the computing device 400 discussed herein) that is local to an entity that services the scientific instrument 1610. For example, the service local computing device 1630 can be local to a manufacturer of the scientific instrument 1610 or to a third-party service company. In one or more embodiments, the service local computing device 1630 can communicate with the scientific instrument 1610, the user local computing device 1620, and/or the remote computing device 1640 (e.g., via a direct communication pathway 1608 or via multiple "indirect" communication pathways 1608, as discussed above) to receive data regarding the operation of the scientific instrument 1610, the user local computing device 1620, and/or the remote computing device 1640 (e.g., the results of self-tests of the scientific instrument 1610, calibration coefficients used by the scientific instrument 1610, the measurements of sensors associated with the scientific instrument 1610, etc.). In one or more embodiments, the service local computing device 1630 can communicate with the scientific instrument 1610, the user local computing device 1620, and/or the remote computing device 1640 (e.g., via a direct communication pathway 1608 or via multiple "indirect" communication pathways 1608, as discussed above) to transmit data to the scientific instrument 1610, the user local computing device 1620, and/or the remote computing device 1640 (e.g., to update programmed instructions, such as firmware, in the scientific instrument 1610, to initiate the performance of test or calibration sequences in the scientific instrument 1610, to update programmed instructions, such as software, in the user local computing device 1620 or the remote computing device 1640, etc.). A user entity of the scientific instrument 1610 can utilize the scientific instrument 1610 or the user local computing device 1620 to communicate with the service local computing device 1630 to report a problem with the scientific instrument 1610 or the user local computing device 1620, to request a visit from a technician to improve the operation of the scientific instrument 1610, to order consumables or replacement parts associated with the scientific instrument 1610, or for other purposes.

The remote computing device 1640 can be a computing device (e.g., in accordance with any of the embodiments of the computing device 400 discussed herein) that is remote from the scientific instrument 1610 and/or from the user local computing device 1620. In one or more embodiments, the remote computing device 1640 can be included in a datacenter or other large-scale server environment. In one or more embodiments, the remote computing device 1640 can include network-attached storage (e.g., as part of the storage device 1604). The remote computing device 1640 can store data generated by the scientific instrument 1610, perform analyses of the data generated by the scientific instrument 1610 (e.g., in accordance with programmed instructions), facilitate communication between the user local computing device 1620 and the scientific instrument 1610, and/or facilitate communication between the service local computing device 1630 and the scientific instrument 1610.

In one or more embodiments, one or more of the elements of the scientific instrument system 1600 illustrated in FIG. 16 can be omitted. Further, in one or more embodiments, multiple ones of various ones of the elements of the scientific instrument system 1600 of FIG. 16 can be present. For example, a scientific instrument system 1600 can include multiple user local computing devices 1620 (e.g., different user local computing devices 1620 associated with different user entities or in different locations). In another example, a scientific instrument system 1600 can include multiple scientific instruments 1610, all in communication with service local computing device 1630 and/or a remote computing device 1640; in such an embodiment, the service local computing device 1630 can monitor these multiple scientific instruments 1610, and the service local computing device 1630 can cause updates or other information can be "broadcast" to multiple scientific instruments 1610 at the same time. Different ones of the scientific instruments 1610 in a scientific instrument system 1600 can be located close to one another (e.g., in the same room) or farther from one another (e.g., on different floors of a building, in different buildings, in different cities, etc.). In one or more embodiments, a scientific instrument 1610 can be connected to an Internet-of-Things (IoT) stack that allows for command and control of the scientific instrument 1610 through a web-based application, a virtual or augmented reality application, a mobile application, and/or a desktop application. Any of these applications can be accessed by a user entity operating the user local computing device 1620 in communication with the scientific instrument 1610 by the intervening remote computing device 1640. In one or more embodiments, a scientific instrument 1610 can be sold by the manufacturer along with one or more associated user local computing devices 1620 as part of a local scientific instrument computing unit 1612.

In one or more embodiments, different ones of the scientific instruments 1610 included in a scientific instrument system 1600 can be different types of scientific instruments 1610; for example, one scientific instrument 1610 can be an EDS device, while another scientific instrument 1610 can be an analysis device that analyzes results of an EDS device. In some such embodiments, the remote computing device 1640 and/or the user local computing device 1620 can combine data from different types of scientific instruments 1610 included in a scientific instrument system 1600.

### Example Operating Environment

FIG. 17 is a schematic block diagram of an operating environment 1700 with which the described subject matter can interact. The operating environment 1700 comprises one or more remote component(s) 1710. The remote component(s) 1710 can be hardware and/or software (e.g., threads, processes, computing devices). In one or more embodiments, remote component(s) 1710 can be a distributed computer system, connected to a local automatic scaling component and/or programs that use the resources of a distributed computer system, via communication framework 1740. Communication framework 1740 can comprise wired network devices, wireless network devices, mobile devices, wearable devices, radio access network devices, gateway devices, femtocell devices, servers, etc.

The operating environment 1700 also comprises one or more local component(s) 1720. The local component(s) 1720 can be hardware and/or software (e.g., threads, processes, computing devices). In one or more embodiments, local component(s) 1720 can comprise an automatic scaling component and/or programs that communicate/use the remote resources 1710 and 1720, etc., connected to a remotely located distributed computing system via communication framework 1740.

One possible communication between a remote component(s) 1710 and a local component(s) 1720 can be in the form of a data packet adapted to be transmitted between two or more computer processes. Another possible communication between a remote component(s) 1710 and a local component(s) 1720 can be in the form of circuit-switched data adapted to be transmitted between two or more computer processes in radio time slots. The operating environment 1700 comprises a communication framework 1740 that can be employed to facilitate communications between the remote component(s) 1710 and the local component(s) 1720, and can comprise an air interface, e.g., interface of a UMTS network, via an LTE network, etc. Remote component(s) 1710 can be operably connected to one or more remote data store(s) 1750, such as a hard drive, solid state drive, subscriber identity module (SIM) card, electronic SIM (eSIM), device memory, etc., that can be employed to store information on the remote component(s) 1710 side of communication framework 1740. Similarly, local component(s) 1720 can be operably connected to one or more local data store(s) 1730, that can be employed to store information on the local component(s) 1720 side of communication framework 1740.

### Example Computing Environment

In order to provide additional context for various embodiments described herein, FIG. 18 and the following discussion are intended to provide a brief, general description of a suitable computing environment 1800 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform tasks or implement abstract data types. Moreover, the methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

Referring still to FIG. 18, the example computing environment 1800 which can implement one or more embodiments described herein includes a computer 1802, the computer 1802 including a processing unit 1804, a system memory 1806 and a system bus 1808. The system bus 1808 couples system components including, but not limited to, the system memory 1806 to the processing unit 1804. The processing unit 1804 can be any of various commercially available processors. Dual microprocessors and other multi processor architectures can also be employed as the processing unit 1804.

The system bus 1808 can be any of several types of bus structure that can further interconnect to a memory bus (with or without a memory controller), a peripheral bus, and a local bus using any of a variety of commercially available bus architectures. The system memory 1806 includes ROM 1810 and RAM 1812. A basic input/output system (BIOS) can be stored in a non-volatile memory such as ROM, erasable programmable read only memory (EPROM), EEPROM, which BIOS contains the basic routines that help to transfer information between elements within the computer 1802, such as during startup. The RAM 1812 can also include a high-speed RAM such as static RAM for caching data.

The computer 1802 further includes an internal hard disk drive (HDD) 1814 (e.g., EIDE, SATA), and can include one or more external storage devices 1816 (e.g., a magnetic floppy disk drive (FDD) 1816, a memory stick or flash drive reader, a memory card reader, etc.). While the internal HDD 1814 is illustrated as located within the computer 1802, the internal HDD 1814 can also be configured for external use in a suitable chassis (not shown). Additionally, while not shown in computing environment 1800, a solid-state drive (SSD) could be used in addition to, or in place of, an HDD 1814.

Other internal or external storage can include at least one other storage device 1820 with storage media 1822 (e.g., a solid-state storage device, a nonvolatile memory device, and/or an optical disk drive that can read or write from removable media such as a CD-ROM disc, a DVD, a BD, etc.). The external storage 1816 can be facilitated by a network virtual machine. The HDD 1814, external storage device 1816 and storage device (e.g., drive) 1820 can be connected to the system bus 1808 by an HDD interface 1824, an external storage interface 1826 and a drive interface 1828, respectively.

The drives and their associated computer-readable storage media provide nonvolatile storage of data, data structures, computer-executable instructions, and so forth. For the computer 1802, the drives and storage media accommodate the storage of any data in a suitable digital format. Although the description of computer-readable storage media above refers to respective types of storage devices, other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1812, including an operating system 1830, one or more application programs 1832, other program modules 1834 and program data 1836. All or portions of the operating system, applications, modules, and/or data can also be cached in the RAM 1812. The systems and methods described herein can be implemented utilizing various commercially available operating systems or combinations of operating systems.

Computer 1802 can optionally comprise emulation technologies. For example, a hypervisor (not shown) or other intermediary can emulate a hardware environment for operating system 1830, and the emulated hardware can optionally be different from the hardware illustrated in FIG. 18. In such an embodiment, operating system 1830 can comprise one virtual machine (VM) of multiple VMs hosted at computer 1802. Furthermore, operating system 1830 can provide runtime environments, such as the Java runtime environment or the .NET framework, for applications 1832. Runtime environments are consistent execution environments that allow applications 1832 to run on any operating system that includes the runtime environment. Similarly, operating system 1830 can support containers, and applications 1832 can be in the form of containers, which are lightweight, standalone, executable packages of software that include, e.g., code, runtime, system tools, system libraries and settings for an application.

Further, computer 1802 can be enabled with a security module, such as a trusted processing module (TPM). For instance, with a TPM, boot components hash next in time boot components, and wait for a match of results to secured values, before loading a next boot component. This process can take place at any layer in the code execution stack of computer 1802, e.g., applied at the application execution level or at the operating system (OS) kernel level, thereby enabling security at any level of code execution.

A user entity can enter commands and information into the computer 1802 through one or more wired/wireless input devices, e.g., a keyboard 1838, a touch screen 1840, and a pointing device, such as a mouse 1842. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera, a gesture sensor input device, a vision movement sensor input device, an emotion or facial detection device, a biometric input device, e.g., fingerprint or iris scanner, or the like. These and other input devices are often connected to the processing unit 1804 through an input device interface 1844 that can be coupled to the system bus 1808, but can be connected by other interfaces, such as a parallel port, an IEEE 1394 serial port, a game port, a USB port, an IR interface, a BLUETOOTH^{®} interface, etc.

A monitor 1846 or other type of display device can also be connected to the system bus 1808 via an interface, such as a video adapter 1848. In addition to the monitor 1846, a computer typically includes other peripheral output devices (not shown), such as speakers, printers, etc.

The computer 1802 can operate in a networked environment using logical connections via wired and/or wireless communications to one or more remote computers, such as a remote computer 1850. The remote computer 1850 can be a workstation, a server computer, a router, a personal computer, portable computer, microprocessor-based entertainment appliance, a peer device or other common network node, and typically includes many or all of the elements described relative to the computer 1802, although, for purposes of brevity, only a memory/storage device 1852 is illustrated. The logical connections depicted include wired/wireless connectivity to a local area network (LAN) 1854 and/or larger networks, e.g., a wide area network (WAN) 1856. Such LAN and WAN networking environments are commonplace in offices and companies, and facilitate enterprise-wide computer networks, such as intranets, all of which can connect to a global communications network, e.g., the Internet.

When used in a LAN networking environment, the computer 1802 can be connected to the local network 1854 through a wired and/or wireless communication network interface or adapter 1858. The adapter 1858 can facilitate wired or wireless communication to the LAN 1854, which can also include a wireless access point (AP) disposed thereon for communicating with the adapter 1858 in a wireless mode.

When used in a WAN networking environment, the computer 1802 can include a modem 1860 or can be connected to a communications server on the WAN 1856 via other means for establishing communications over the WAN 1856, such as by way of the Internet. The modem 1860, which can be internal or external and a wired or wireless device, can be connected to the system bus 1808 via the input device interface 1844. In a networked environment, program modules depicted relative to the computer 1802 or portions thereof, can be stored in the remote memory/storage device 1852. The network connections shown are example and other means of establishing a communications link between the computers can be used.

When used in either a LAN or WAN networking environment, the computer 1802 can access cloud storage systems or other network-based storage systems in addition to, or in place of, external storage devices 1816 as described above. Generally, a connection between the computer 1802 and a cloud storage system can be established over a LAN 1854 or WAN 1856 e.g., by the adapter 1858 or modem 1860, respectively. Upon connecting the computer 1802 to an associated cloud storage system, the external storage interface 1826 can, with the aid of the adapter 1858 and/or modem 1860, manage storage provided by the cloud storage system as it would other types of external storage. For instance, the external storage interface 1826 can be configured to provide access to cloud storage sources as if those sources were physically connected to the computer 1802.

The computer 1802 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a defined structure as with an existing network or simply an ad hoc communication between at least two devices.

### Additional Information

The embodiments described herein can be directed to one or more of a system, a method, an apparatus and/or a computer program product at any possible technical detail level of integration. The computer program product can include a computer readable storage medium (or media) having computer readable program instructions thereon for causing a processor to carry out aspects of the one or more embodiments described herein. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a superconducting storage device and/or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, is not to be construed as being transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein can be downloaded to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more embodiments, electronic circuitry including, for example, programmable logic circuitry, field-programmable gate arrays (FPGA) and/or programmable logic arrays (PLA) can execute the computer readable program instructions by utilizing state information of the computer readable program instructions to personalize the electronic circuitry, in order to perform aspects of the one or more embodiments described herein.

Aspects of the one or more embodiments described herein are described with reference to flowchart illustrations and/or block diagrams of methods, apparatus (systems), and computer program products according to one or more embodiments described herein. It will be understood that each block of the flowchart illustrations and/or block diagrams, and combinations of blocks in the flowchart illustrations and/or block diagrams, can be implemented by computer readable program instructions. These computer readable program instructions can be provided to a processor of a general-purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computer-implementable methods and/or computer program products according to one or more embodiments described herein. In this regard, each block in the flowchart or block diagrams can represent a module, segment and/or portion of instructions, which comprises one or more executable instructions for implementing the specified logical function. In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter has been described above in the general context of computer-executable instructions of a computer program product that runs on a computer and/or computers, those skilled in the art will recognize that the one or more embodiments herein also can be implemented at least partially in parallel with one or more other program modules. Generally, program modules include routines, programs, components and/or data structures that perform particular tasks and/or implement particular abstract data types. Moreover, the aforedescribed computer-implemented methods can be practiced with other computer system configurations, including single-processor and/or multiprocessor computer systems, mini-computing devices, mainframe computers, as well as computers, hand-held computing devices (e.g., PDA, phone), and/or microprocessor-based or programmable consumer and/or industrial electronics. The illustrated aspects can also be practiced in distributed computing environments in which tasks are performed by remote processing devices that are linked through a communications network. However, one or more, if not all aspects of the one or more embodiments described herein can be practiced on stand-alone computers. In a distributed computing environment, program modules can be located in both local and remote memory storage devices.

As used in this application, the terms "component," "system," "platform" and/or "interface" can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form. As used herein, the terms "example" and/or "exemplary" are utilized to mean serving as an example, instance, or illustration. For the avoidance of doubt, the subject matter described herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

As it is employed in the subject specification, the term "processor" can refer to substantially any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nano-scale architectures such as, but not limited to, molecular and quantum-dot based transistors, switches and/or gates, in order to optimize space usage and/or to enhance performance of related equipment. A processor can be implemented as a combination of computing processing units.

Herein, terms such as "store," "storage," "data store," data storage," "database," and substantially any other information storage component relevant to operation and functionality of a component are utilized to refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory and/or nonvolatile random-access memory (RAM) (e.g., ferroelectric RAM (FeRAM). Volatile memory can include RAM, which can act as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein are intended to include, without being limited to including, these and/or any other suitable types of memory.

What has been described above includes mere examples of systems and computer-implemented methods. It is, of course, not possible to describe every conceivable combination of components and/or computer-implemented methods for purposes of describing the one or more embodiments, but one of ordinary skill in the art can recognize that many further combinations and/or permutations of the one or more embodiments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the various embodiments can use the phrases "an embodiment," "various embodiments," "one or more embodiments" and/or "some embodiments," each of which can refer to one or more of the same or different embodiments.

The descriptions of the various embodiments have been presented for purposes of illustration but are not intended to be exhaustive or limited to the embodiments described herein. Many modifications and variations will be apparent to those of ordinary skill in the art without departing from the scope and spirit of the described embodiments. The terminology used herein was chosen to best explain the principles of the embodiments, the practical application and/or technical improvement over technologies found in the marketplace, and/or to enable others of ordinary skill in the art to understand the embodiments described herein.

## Claims

1. A computer-implemented method, comprising:
executing, by a system operatively coupled to a processor, a comparison of first spectrum data to second spectrum data;
based on the comparison, generating, by the system, a spectrum similarity score describing a level of similarity of the first spectrum data to the second spectrum data;
based on the comparison, associating, by the system, a first secondary property corresponding to the first spectrum data with the second spectrum data or associating, by the system, a second secondary property corresponding to the second spectrum data with the first spectrum data; and
generating, by the system, a grouping of spectral data comprising the first spectrum data and the second spectrum data based on the spectrum similarity score and on the associating.

2. The computer-implemented method of claim 1, wherein the grouping of spectral data comprises a dataset or data employed to generate a visualization.

3. The computer-implemented method of claim 1 or claim 2, wherein the associated one of the first secondary property or the second secondary property is defined by identification metadata associated with the first spectrum data or the second spectrum data.

4. The computer-implemented method of claim 1, claim 2 or claim 3, further comprising:
generating, by the system, the spectrum similarity score describing a comparison of a first mass to charge ratios of ions of the first spectrum data to a second mass to charge ratios of ions of the second spectrum data.

5. The computer-implemented method of any preceding claim, wherein the first spectrum data is a first unknown spectrum data, and wherein the second spectrum data is a second unknown spectrum data.

6. The computer-implemented method of any preceding claim, wherein the associated one of the first secondary property or the second secondary property comprises one or more, but not limited to, chemical compound use class, substructural similarity, fragmentation kinetics breakdown curves, optimal energy, peak counts, chemical structure descriptive class or superclass, toxicological characteristics, physico-chemical characteristics, metabolic pathway, enzymatic reactions, biological reactions, enzymes or catalysts, or organisms or tissues.

7. A computer program facilitating a process for generation of one or more spectral data groupings, the computer program comprising program code which, when executed by a processor, causes the processor to carry out the method steps of any of the preceding claims.

8. A computer program product upon which is stored or embodied the computer program of claim 7.

9. A system comprising a memory that stores the computer program of claim 7 and a processor that executes the program code stored in the memory.
